# EUROPEAN PATENT SPECIFICATION

(11) **EP 3 914 388 B1**
(45) Date of publication and mention of the grant of the patent: **03.07.2024**
(21) Application number: 20707930.2
(22) Date of filing: 23.01.2020
(51) Int. Cl.: B01L 3/00, G01N 1/40, B01D 61/14, C12M 1/00, C12M 1/26, B01D 63/08, B01D 67/00, A61M 5/165

(54) **METHODS AND APPARATUS TO SELECTIVELY EXTRACT CONSTITUENTS FROM BIOLOGICAL SAMPLES**
VERFAHREN UND VORRICHTUNG ZUR SELEKTIVEN EXTRAKTION VON BESTANDTEILEN AUS BIOLOGISCHEN PROBEN
PROCÉDÉS ET APPAREIL POUR EXTRAIRE SÉLECTIVEMENT DES CONSTITUANTS D'ÉCHANTILLONS BIOLOGIQUES

(30) Priority: 25.01.2019 US 201962796757 P; 27.09.2019 US 201962906822 P
(43) Date of publication of application: 01.12.2021
(73) Proprietor: Becton, Dickinson and Company, Franklin Lakes, NJ 07417-1800 (US)
(72) Inventor: XU, Qihua, Cary, NC 27519 (US); WEIDEMAIER, Kristin, Research Triangle Park, NC 27614 (US); SALOMON, Jon, E., Stewartstown PA 17363 (US); LASTOVICH, Alexander, G., Raleigh, NC 27614 (US); FALLOWS, Eric, A., Apex, NC 27502 (US); CONNELL, Sean, Gilbert, AZ 85233 (US); HERR, Joshua, Phoenix, MD 21131 (US); WOLFGANG, Meghan, Pittsboro, NC 27312 (US); BRASCH, Michael, A., Gaithersburg, MD 20882 (US); MOORE, Richard, L., Glenville, PA 17329 (US); SEBBA, David, S., Sparks, MD 21152-9212 (US); CLAVIJO, Cristian, Lehi, UT 84043 (US); NG, Shirley, Baltimore, MD 21218-2421 (US); ABBOT, Richard, Research Triangle Park, NC 27614 (US); PAPP, Alexander, Adam, Morrisville, NC 27560 (US); FU, Henry, Li-wei, Durham, NC 27713 (US); AUSTIN, Caitlin, Marie, Sparks, MD 21152-9212 (US); DOWLING, Sean, Patrick, Morrisville, NC 27560 (US); JOYCE, Owen, Lewis, Morrisville, NC 27560 (US); KIPLINGER, Michael, L., Morrisville, NC 27560 (US); CARPENTER, William, Kevin, Morrisville, NC 27560 (US)
(74) Representative: dompatent von Kreisler Selting Werner - Partnerschaft von Patent- und Rechtsanwälten mbB
(86) International application number: PCT/US2020/014758
(87) International publication number: WO 2020/154484

(56) References cited:
- WO-A1-92/04970
- WO-A1-2017/082990
- WO-A1-2018/129429
- US-A1- 2007 246 406
- US-A1- 2012 085 648
- US-A1- 2014 190 903
- Kelly William ET AL: "Understanding and modeling alternating tangential flow filtration for perfusion cell culture", Biotechnology Progress, vol. 30, no. 6, 6 August 2014 (2014-08-06) , pages 1291-1300, XP055957373, ISSN: 8756-7938, DOI: 10.1002/btpr.1953

## Description

### RELATED APPLICATIONS

This application claims priority from US Provisional Application Serial No. 62/796,757 filed January 25, 2019. This application also claim priority from US Provisional Application Serial No. 62/906,822 filed September 27, 2019.

### FIELD OF THE TECHNOLOGY

The present technology relates to methods and apparatus that selectively extract constituents from biological samples for further analysis or diagnostic purposes including, for example, tangential flow extraction, single stage extraction and multiple stage extraction, using separation mechanisms such as membranes. Certain exemplary embodiments of the extraction technology may involve bandpass filtering using tangential flow filtering/extraction to separate components of biological samples such as blood samples (diluted or undiluted), positive blood cultures, urine samples, etc. which may facilitate direct and rapid identification and/or antimicrobial susceptibility testing.

### BACKGROUND OF THE TECHNOLOGY

Biological samples (i.e. samples pertaining to life or living organisms) are analyzed in a multitude of contexts. Biological samples are complex mixtures of cells, proteins, vesicles and other components. These components vary among themselves in terms of structure, size, composition, etc. It is often desirable to selectively analyze discrete components of biological samples for research, diagnosis, epidemiology, treatment, etc. For example, the presence or absence of bacterial or fungal pathogens in biological samples can be used to diagnose conditions such as sepsis or urinary tract infections in a patient/subject. As another example, the type and distribution of extracellular vesicles in a sample component (e.g., cells) may provide information useful in the detection and treatment of cancer. However, detection and analysis of discrete components (e.g. pathogens, cells, or extracellular vessels) in biological samples such as blood is complicated by the presence of the other components in the biological samples, which may interfere with the methods designed to detect a component of interest. The ability to isolate and/or concentrate the component of interest and separate it from other components of the biological sample is therefore of critical importance.

Methods for such isolation exist but such methods are not completely desirable. For example, centrifugation and wash methods are labor-intensive and subject to user-variability. Filtration methods have tendency to lose the component of interest to interactions with the filter surface(s). Microbiology plating methods allow the component of interest (e.g. bacteria) to be grown and separated, but require long replication times.

Methods of isolation/concentration that require long wait times are particularly problematic. One important application where there is a need for improved sample processing methods is sepsis detection. Patients admitted to an Intensive Care Unit (ICU) with severe sepsis have a 39.8% risk of death. Therefore, rapidly identifying the responsible pathogen and appropriate antibiotic treatment is imperative for improving patient survivability. Current methods for determining the identity (ID) and antimicrobial susceptibility testing (AST) of pathogens recovered from a positive blood culture (PBC) require the specimen to be isolated from the blood background. The standard approach is to subculture an aliquot of the PBC to isolate and, in some embodiments, further concentrate the pathogens. This subculturing step can delay the time to results by approximately 18-24 hours.

Therefore, it is desirable to implement improved techniques and devices for evaluating components of interest in biological samples that can isolate and evaluate such components quickly. Such techniques may improve the efficiency and/or accuracy of testing.
US 2014/190903 A1 discloses a system for particle filtration.
WO 92/04970 A1 discloses an improved tangential filtration apparatus.
US 2012/085648 A1 discloses a microfluidic fluid separator.

### SUMMARY OF THE TECHNOLOGY

The subject matter of the invention is defined by independent claims 1 and 10.

Examples of the technology disclosed herein can provide a process that may effectively separates pathogens from the blood background without subculturing and may thereby facilitate rapid ID/AST. The sample preparation may include: i) separation of the target pathogen from the sample (e.g. isolation due to size exclusion); ii) increasing the concentration of the target pathogen; or iii) purification of the target pathogen by washing out the impurities while retaining the target. In one example, the target pathogen is a bacterium that is isolated and collected for identification testing or susceptibility testing. Such testing can be paired with automated analysis such as digital imaging of plated cultures over time for the detection of microbial growth thereon or light testing of target pathogen cultures.

Another aspect of the present technology is to implement extraction of components of a biological sample for concentrating a desired constituent.

Another aspect of the present technology is to facilitate biological sample analysis without requiring a subculture step to obtain a sample with a sufficient amount of biomass/colony mass and/or with sufficient purity.

In one embodiment, an apparatus for extracting constituents from a biological sample for concentrating a desired constituent of the biological sample includes a plurality of membrane stages, wherein each of the plurality of membrane stages has a membrane having a first side, a second side, a first end and a second end, the membrane having a characteristic to selectively permit one or more constituents of the biological sample to pass through the membrane from the first side to the second side while retaining other constituents of the biological sample at the first side. The membrane also has an input chamber having an inlet proximate to the first end and an outlet proximate to the second end, the input chamber configured at the first side of the membrane to permit (a) a tangential flow of the biological sample along a first surface of the membrane at the first side from the inlet to the outlet, and (b) a trans-membrane passing of the one or more constituents of the biological sample from the first side to the second side. The apparatus also has an output chamber configured at a second surface of the membrane at the second side and configured to receive the one or more constituents of the biological sample that pass through the membrane. The membrane stages are collectively configured to isolate a desired passband of constituents of the biological sample.

In another embodiment, there is a single stage tangential flow membrane device that has an input chamber having first and second outlets. The device also has an output chamber having a second outlet. The filtration membrane defines a tangential flow path that separates the first chamber from the second chamber and also has a recirculating flow path for directing the sample to pass over the filtration membrane a plurality of times. A component of interest is recovered from the portion of the sample that is retained by the filtration membrane in the input chamber (i.e. the retentate).

In another embodiment, the single stage tangential flow membrane device is configured as a cartridge. The cartridge has a port that receives a sample container, and the port is fluidically coupled to a sample chamber in the cartridge. The sample chamber is fluidically coupled to the first inlet of the input chamber. The first outlet of the input chamber is fluidically coupled to the sample chamber such that retentate from the filtration membrane is recirculated back to the sample chamber.

A method for extracting constituents from a biological sample is described herein. According to the method, in a first extracting process, one or more constituents of the biological sample are passed through, by a pressure force, a first membrane from a first side of the first membrane to a second side of the first membrane while retaining other constituents of the biological sample at the first side of the first membrane and while generating a tangential flow of the biological sample along a surface of the first membrane at the first side of the first membrane. In a second extracting processes one or more additional constituents of the one or more constituents are passed through, using a pressure source, a second membrane from a first side of the second membrane to a second side of the second membrane while retaining some of the one or more constituents at the first side of the second membrane and while generating a tangential flow of the one or more constituents along a surface of the second membrane at the first side of the second membrane. According to the method the first extracting process and the second extracting process isolate, purify and/or concentrate a desired passband of constituents of the biological sample from other constituents of the biological sample.

In another embodiment, a method for isolating pathogens from a positive blood culture is disclosed. According to the method, a blood culture suspected to contain target pathogens is flown into a sample chamber. The blood culture is flown into a filtration device. The filtration device comprises a filtration membrane that separates a first chamber from a second chamber. The blood culture is flown tangentially across the filtration membrane such that the target pathogens, if present in the positive blood culture, flow across the filtration membrane and through a first outlet of the first chamber as retentate. Portions of the positive blood culture having a particle size less than a predetermined threshold flow through the filtration membrane and into the second chamber. The retentate is flown through the first outlet of the first chamber to recirculate over the filtration membrane whereby a further portion of the sample permeates through the filtration membrane. After recirculating the retentate a predetermined number of times, the retentate is washed by combining the retentate with a wash buffer. The washed retentate in a vial for downstream testing to determine the presence or absence of target pathogens in the retentate.

Other features of the technology will be apparent from consideration of the information contained in the following detailed description, drawings and claims.

### BRIEF DESCRIPTION OF THE DRAWINGS

The present technology is illustrated by way of example, and not by way of limitation, in the figures of the accompanying drawings, in which like reference numerals refer to similar elements including:
FIG. 1 illustrates one example of a membrane device suitable for use in some versions of the present technology;
FIG. 2 is another example of a membrane device that is configured with a vacuum chamber to promote tangential flow filtration of the present technology;
FIG. 3 is an example membrane device that is configured with syringe type plungers to promote tangential flow filtration of the present technology;
FIG. 4 illustrates a radial version of an example membrane device(s) that is configured to promote tangential flow filtration of the present technology;
FIG. 5 illustrates a recirculating pump version of an example membrane device that is configured to promote tangential flow filtration of the present technology;
FIG. 6A illustrates steps of an example filtration methodology with membrane filtration of a blood sample containing *E*. *coli,* permitting multi-pass concentration of the bacteria from the blood sample;
FIG. 6B is a graph illustrating an increase in diagnostic speed when the bacteria in the sample is isolated with a tangential flow filtration process of the present technology;
FIGs. 7A and 7B illustrate further examples of membrane devices that isolate exosomes in a sample using a multi-stage process;
FIG. 8 is a graph illustrating dynamic light scattering data of retentate for extracted exosome particles using a two-stage process as described herein;
FIG. 9 is a graph illustrating signal intensity of the filtrate in which exosome particles are extracted using a two-stage process as described herein;
FIG. 10 is a graph illustrating a comparison of *E*. *coli* recovery rates from BACTEC lytic bottles and BACTEC standard bottles using a single stage filter for separating *E. coli* from the sample;
FIG. 11 is an illustration of a low pass membrane tangential flow filtration process of an example of the present technology;
FIG. 12 illustrates steps of an example methodology for multistage tangential flow filtration using membrane devices of the present technology;
FIG. 13 illustrates components and processing for a stage of a tangential flow filtration process using an example membrane device of the present technology;
FIGs. 14A-K describe a fluidic circuit for a disposable cartridge that performs tangential flow filtering according to one embodiment;
FIG. 15 illustrates identification and antibiotic Susceptibility testing results for bacteria isolated from a contrived positive blood culture using a single stage tangential flow filtration process;
FIG. 16 illustrates identification and antibiotic susceptibility testing results for bacteria isolated from contrived positive blood culture samples using a two-stage tangential flow filtration process;
FIG. 17 illustrates how the method and device described herein can be integrated into known workflows; compares the time required to evaluate the bacteria in a positive blood culture using the method and system described herein compared to other conventional methods;
FIG. 18 illustrates a benchtop instrument for removing or isolating or concentrating pathogens from a positive blood culture;
FIGs. 19A-F illustrate one example of a workflow for the benchtop instrument illustrated in FIG. 18;
FIGs. 20A-D illustrate alternative workflows and configurations of the benchtop instrument illustrated in FIG. 18.
FIGs. 21A-F illustrate a potential workflow process of one embodiment of the disposables and benchtop instrument;
FIGs. 22A-B illustrate two potential methods of introducing the positive blood culture to the system; and
FIGs. 23A-C illustrate potential methods for suppling any required reagents or buffers.

### DETAILED DESCRIPTION

Some embodiments of the present biological extraction technologies may implement employing tangential flow extraction employing one or more extraction stages.

### Tangential flow extraction principle and concept

The present technology provides apparatus and methods to isolate or purify (and in some embodiments concentrate) particular constituents of interests from biological samples including, for example, blood, urine, saliva and other sample types using membrane-based technologies in combination with tangential flow extraction. Examples of particular constituents of interest can be target analytes, dissociated or circulating tumor cells (CTC), white blood cells (WBC), extracellular vesicles like exosomes, yeast, bacteria and virus, etc. The membrane-based technology can be implemented with tangential flow processing of a biological sample in liquid form. Such a tangential fluid flow may pass (e.g., repeatedly) the sample over a specified membrane surface that has a trans-membrane pressure characteristic that is engineered to allow a target constituent to pass through the membrane as a permeate or to be retained by the membrane as a retentate. Thus, the specified membrane or membrane device may be selectively configured depending on the target and whether the target will be passed or retained. This technique, unlike many other technologies, offers a beneficial opportunity to maintain viability and integrity of vulnerable extracted constituents such as cells, bacteria, virus, etc. that can be compromised by harsh extraction conditions. In addition, tangential flow separation described herein permits efficient recovery of components extracted from the sample, even at low concentrations, because exposure of the components to the membrane surface area is significantly reduced compared to other filtration approaches such as dead-end filtration or pass-through filtration in which the filter is made of fibrous materials. Thus, the separation method and device described herein reduces the risk of target loss due to binding or damage.

In order to achieve extraction of the target constituents, device design, membrane pore size and process parameters are engineered. The target constituent can be retained or passed through the device membrane depending on the objectives of the extraction process. The porous membrane is preferred to have a generally uniform pore size (a characteristic pore size) and smooth surface relative to the particles to be extracted. One such type of membrane is a track etched membrane (TEM) which can be made from polyester, polypropylene, polycarbonate, polyimide etc. Materials suitable for TEMs are well known to those skilled in the art and not described in detail herein. Such membranes are commercially available and can have uniform pore size ranging from nanometer to micrometer size. Additionally, such membranes can be further treated to modify surface properties such as hydrophobicity to reduce membrane fouling or otherwise improve application performance. Such coatings and treatments are well known to the skilled person and not described in detail herein. The system may preferably be designed to have a correlated tangential flow rate and trans-membrane pressure. The tangential flow rate and trans-membrane pressure are chosen to: i) prevent fouling of the membrane due to phenomena such as cake layer formation or pore clogging with particulate; and ii) optimize the sample processing time without introducing undesired shear or lysing of cellular component. Such flow rate and pressure characteristic selection can allow the target particle size to stay in the retentate phase or pass through the selected membrane as the permeate phase when paired with the proper selection of the membrane pore size for the target constituents to be extracted.

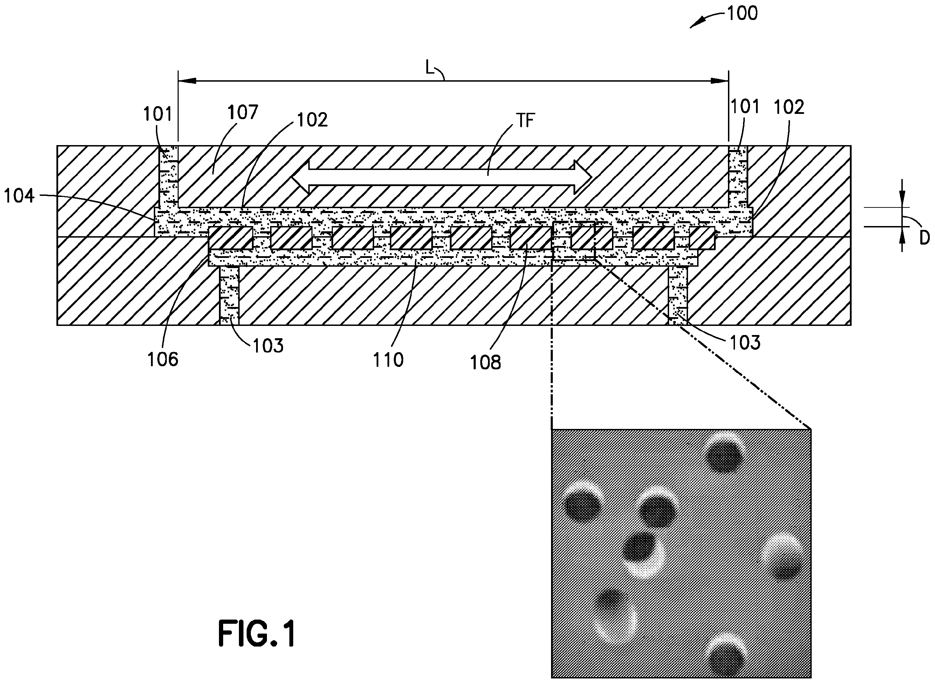

An extraction process is illustrated with reference to the device depicted in FIG. 1, where fundamental tangential flow filtration in a membrane device 100 can serve to process the sample 102. The membrane device 100 may typically include an input or upper chamber 104 and an output or lower chamber 106 that are formed and joined by a porous membrane 108 interposed therebetween. The upper chamber 104 will typically have sample inlet/outlet(s) 101 to/from the inlet chamber for inflow and outflow of the sample with respect to the inlet chamber 104. The output or lower chamber may have outlet(s) 103 for outflow of the smaller constituents 110 and the liquid carrying the smaller constituents that passes through the membrane 108. The inlet/outlet(s) are typically at or near ends of the membrane to promote or maximize the tangential flow (TF) area. Thus, the biological sample flows tangentially along the membrane surface and smaller constituent 110 matter and liquid can pass through the membrane and be isolated from the rest of the fluid and other constituents of the sample 102 that do not pass through the membrane.

Optionally, the tangential flow (i.e., along the membrane surface) and/or trans-membrane flow (i.e., across the membrane) can be driven by a pressure differential; i.e., by either a positive pressure source and/or a negative pressure source (e.g., a vacuum source). The tangential flow can be one directional flow or reciprocating flow (where fluid flow reverses its direction in order to allow fluid flow over the same membrane surface multiple times to complete the filtration process). Multiple passes of the sample over the membrane provides a number of potential benefits including the reduction of the required membrane area. In some implementations, the tangential flow can be powered by a circulation or pressure pump for driving tangential flow in one direction. In some such cases, the sample will flow multiple times along the surface of the membrane such as where the flow path employs a loop or circuit and a pump circulates or re-circulates the sample fluid in the loop/circuit. In other examples, the sample flows multiple times in different directions along the surface of the membrane such as where the flow path is terminated (e.g., non-looped) and it is coupled to one reversible pump (i.e., bi-directional) or a plurality of alternating uni-directional pumps that achieve the reciprocating tangential flow. In some examples, such pumps/vacuums may optionally be implemented by one or more syringes, which may be manually or automatically operated. Other types of pumps/vacuums may optionally be implemented, or appropriate valving configurations using a single power source deployed, thereby achieving the desired flow. Pumps/vacuums that can be used to drive the flow of the sample across the membrane are well known to one skilled in the art and not described in detail herein.

In some versions, a pressurized container and/or depressurized container can be coupled to the inlet/outlets (101, 103) of the chamber(s) to provide the force for the tangential flow. For example, such a coupling may be at or near opposing ends of the membrane to induce the tangential flow along one surface of the membrane from end to end (i.e., in one chamber). The chamber can be configured to induce flow along the membrane such as when the chamber is bounded by a wall structure 107 to form a narrow gap proximate to and along the surface of the membrane. The tangential flow occurs within the gap. Such as gap may beneficially be longer in dimension along the length (L) of the membrane when compared to the depth (D) of the gap between the membrane and a surface of the wall structure 107 that forms the gap.

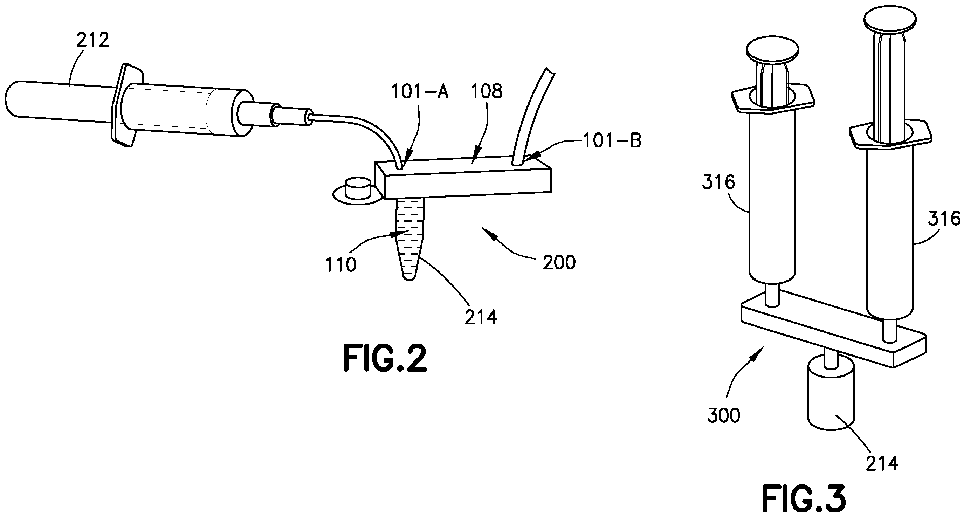

As illustrated in FIG. 2 a depressurized version of the container 212 may be an evacuated collection tube (e.g. the BD Vacutainer^{®}) that may be coupled to an inlet/outlet 101-A to the upper chamber near a proximate end, such as via a conduit. Optionally, a needle, such as for drawing blood, may be coupled at or near an opposing end to an inlet/outlet 101-B, via a conduit. A collection container 214, such as a vial that may fixed or removable from the membrane device 200, may collect the liquid and constituent 110 (permeate) that passes through the membrane 108. In the example of FIG. 3, multiple plunger devices 316 (e.g., syringes) serve to force a reciprocating flow of sample in the membrane device 300 to induce particular sample constituents/analytes across the internal membrane for collection in the collection container 214.

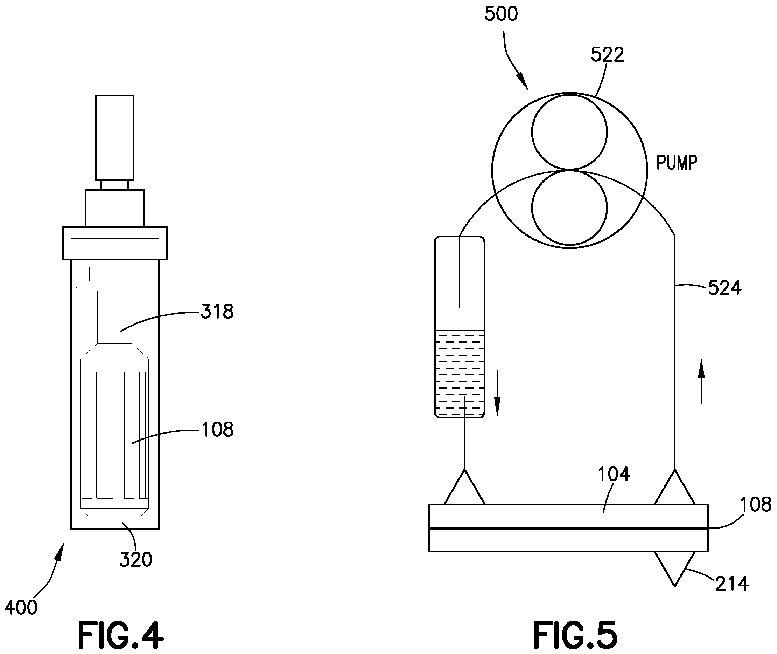

Alternatively, the tangential flow can also be created by moving the membrane. One such example is illustrated in FIG. 4. The device 400 may be a rotational spindle 318 (e.g., a hollow cylindrical structure) with an internal chamber and a porous membrane 108 on outer surface of the spindle 318. The spindle 318 may be rotatably mounted inside a cylindrical housing 320. A biological sample such a blood may be introduced to the spindle 318 and the cylindrical housing 320. The tangential flow is created by rotating the housing 320 while keeping the spindle 318 cylindrical membrane insert static or vice versa. A trans membrane flow pressure can be provided to induce smaller constituents from the cylindrical housing 320 to pass through the membrane 108 into the hollow chamber of the spindle as the spindle rotates relative to the cylindrical housing. In such a case, the hollow portion of the spindle serves as a collection container for the permeate. An example of such a device is illustrated in US Patent Publication No. 2018/0056243 published March 1, 2018 and entitled "Plasma Extractor,".

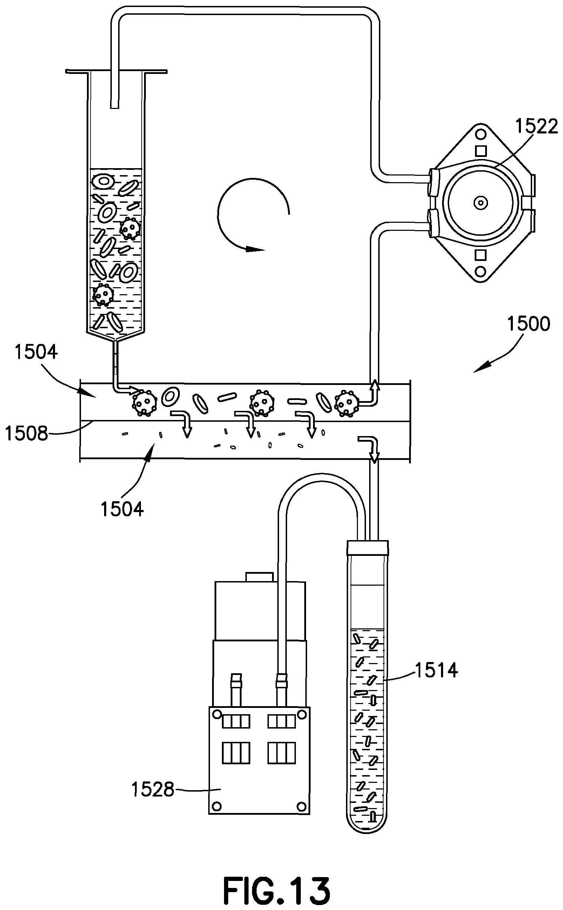

In the example of FIG. 5, a recirculating pump 522 (e.g., a peristaltic pump) recirculates the biological sample through a loop 524 (e.g., conduit including upper chamber 104 of the membrane device 500) tangentially along one surface of the internal membrane 108. The conduit loop permits re-circulation for multiple passes along the internal membrane 108. In a similar configuration shown in FIG. 13, the recirculating pump 1522 recirculates the biological sample through a loop including the input chamber 1504 of the membrane device 1500 so that the sample repeatedly flows tangentially along the membrane 1508. Such a pump may also provide a trans-membrane pressure. A vacuum pump 1528 may also be coupled to the output chamber of the membrane device 1500 and may provide a trans-membrane pressure, drawing the permeate through the membrane and into a collection container 1514. The recirculation of sample allows for removal of additional/unwanted (i.e., background) components from the sample, thereby improving the degree and extent of isolation, purification and/or concentration of the pathogens in the sample from other sample constituents. Recirculation can be deployed in either the single stage or multistage configurations of the tangential flow membrane device described herein.

### A. Single stage extraction process

As previously mentioned, the membrane device 100 can be implemented for a single stage extraction such as where the sample tangentially flows along a single membrane or multiple membranes with the same filter characteristics (e.g., same pore size). Such a process may be implemented for bacteria recovery and concentration from fresh whole blood, urine, cerebrospinal fluid, saliva, respiratory samples, and wounds, as well as other sample types. Example processes are described herein.

### (1) Bacteria Recovery and Concentration from Fresh Whole Blood

As previously mentioned, fast results of bacteria identification/detection and antibiotics susceptibility testing are desired to improve treatment efficiency. Conventional test procedures involve a long incubation time and complex sample purification steps due to low bacteria concentration in the original samples such as blood or urine. Concentrating the bacteria directly from the patient sample delivers quicker results by improving the detectability or reducing the incubation time.

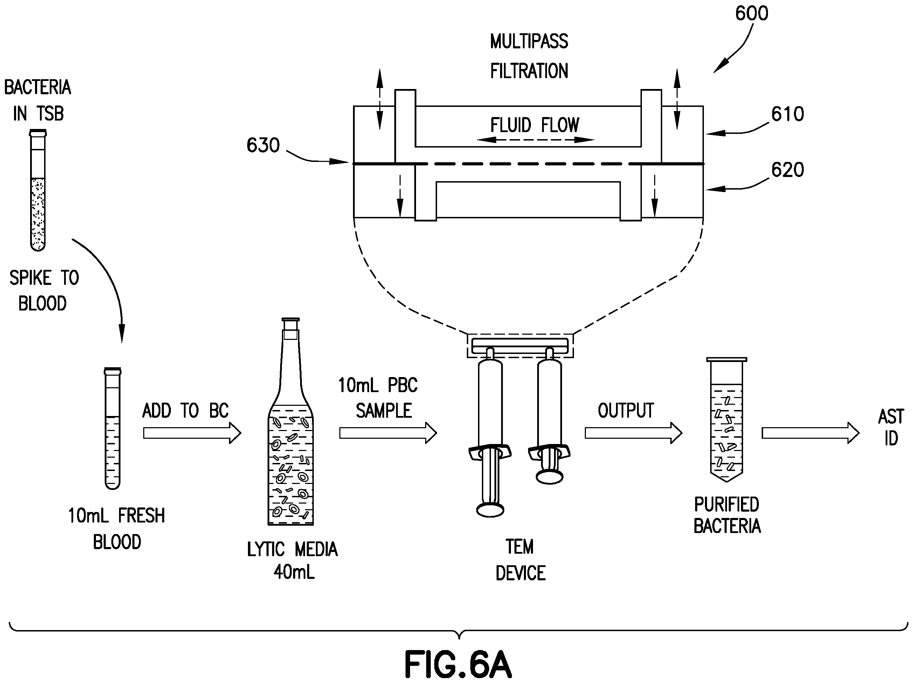

Tangential flow extraction can serve as a concentration process using a membrane device 100 and can achieve the enrichment of the bacteria in the original sample, such as in an *E. coli* bacteria recovery test. For example, bacteria may be isolated and, in some embodiments, concentrated from a blood sample. In an example of such a process as illustrated in FIG. 6A, a 10 mL patient blood sample is collected. For test purposes it is spiked with *E. coli.* The sample is diluted using 40 mL BD BACTEC^{™} Lytic lysing media. BACTEC is a trademark of Becton, Dickinson, and Company (BD). The media enables a differential lysis where the blood components are lysed but the bacteria are not lysed. The lysed sample is applied to a tangential flow extraction process where bacteria will be isolated from the liquid medium. The membrane device 600 (or tangential flow extraction device), similar to the previously discussed examples, has a first chamber 610 (e.g., input or upper chamber) and a second chamber 620 (e.g., output or lower chamber) separated by a track etched membrane (TEM) 630. The lysed blood sample flows (repeatedly in a multi-pass process) along the track etched membrane in membrane device 600 in the first chamber (e.g., upper chamber; 104 in FIG. 1) and partial liquid phase flows through the track etched membrane 630 (TEM 108 in FIG. 1) into the second chamber (e.g., lower chamber; 106 in FIG. 1) by applying a trans-membrane pressure. The TEM may have a pore size ranging from about 0.2 to about 2.0 µm or alternatively about 0.4 µm to about 1 µm. Such pore size is selected to retain the bacteria in the retentate phase. The tangential flow rate can be in the range of about 1 to about 20 10 mL/min or alternatively from about 5 mL/min. One skilled in the art will appreciate that the flow velocity across the membrane is affected by the cross-sectional geometry of the chambers. The flow rate range above is based on a chamber geometry of 10 mm x 40 mm x 0.08 mm. In this embodiment, the trans-membrane pressure is in the range of about 0.1 psi (about 0.69 kPa) to about 10 psi (about 68.95 kPa), alternatively in the range of about 0.5 (about 3.45 kPa) to about 6 psi (about 41.4 kPa). Within a brief period of time (e.g., about 5 to about 10 minutes) bacteria is concentrated as volume is reduced. In one example where the sample is diluted (e.g. a blood sample of 10mL diluted to a volume of 50 mL in a lytic media), 10mL of the diluted sample (proportionately about 2mL of blood and 8 mL of media) is reduced to a 0.5 mL volume (i.e. a reduction of about 4:1) in which the bacteria is isolated in a collection chamber coupled to the membrane device. The final volume can be varied based on the target concentration and is generally only limited by the device dead volume and the ability to continue to move fluid as the viscosity increases due to the higher proportion of solid content. In one example, the isolated bacteria sample was plated on an eosin-methylene blue (EMB) plate and incubated overnight. A colony forming unit (CFU) count was used to determine the bacteria concentration and recovery rate.

In one example, a tangential flow filtration system is similar to the membrane device 600 with a first chamber geometry of 10 mm x 40 mm x 0.08 mm. The membrane pore size of 0.4 um was used. The 10 mL diluted blood sample (2 mL whole blood and 8 mL BACTEC media) containing spiked bacteria (*E.coli*) were processed at the tangential flow rate of 10 mL/min with a trans-membrane pressure of approximately 3 psi in a reciprocating flow mode where the fluid flow reverses flow direction multiple times along the membrane surface. The process was stopped when the retentate volume reached to about 0.5mL and the retentate was collected for further bacteria count determination by plating on an eosin-methylene blue (EMB) plate and incubating overnight. Table 1 below shows that the bacteria viability was not affected by the reciprocating multi-pass tangential flow filtration process and showed good recovery. The percent average recovery is calculated based on average recovered bacteria CFU count from TEM devices as referenced to the count from the sample taken directly from the differentially lysed blood solution (centrifuging the blood to collect the bacteria). About 95 to 100% recovery is achieved in this study for bacteria concentration above 03 CFU/mL. At the low spike concentration of 5 CFU/mL input whole blood, the recovery is 82% on average. The loss can be attributed to the loss of bacteria that occurs in the device due to the dead volume or due to a minor loss of viability of a few microorganisms. The loss of just a few organisms in the low spike results in a greater measured loss since each one represents a higher percentage of the total than those in the higher titer samples.

**Table 1 Results of E. coli bacteria recovery test**

| **Sample** | **Spiked E Coli concentration , CFU/mL ^{a}** | **# of TEM device Replicates** | **% Average recovery from TEM device** |
|---|---|---|---|
| 2 mL blood +8 mL BACTEC^{™} BC Lytic | 600 | 4^{b} | 104 |
| 2 mL blood +8 mL BACTEC^{™} BC Lytic | 30 | 4^{c} | 95 |
| 2 mL blood +8 mL BACTEC^{™} BC Lytic | 5 | 6^{c} | 82 |

| | | | |
|---|---|---|---|
| ^{a}Bacteria concentration is based on whole blood volume. Control sample is taken from blood culture bottle. Results are the average of three plate counts. ^{b}Concentrate was plated on three EMB plates per device. ^{c}Concentrate was plated on one EMB plate per device. | | | |

### (2) Bacteria Recovery and Isolation from a Positive Blood Culture

As previously described, rapid bacteria identification and antibiotic susceptibility testing of bacteria are sought to improve treatment efficiency. Conventional test procedures involve a lengthy subculture step after the identification of a positive blood culture to obtain bacteria for identification and antibiotic susceptibility testing. Isolating and washing the bacteria directly from the positive blood culture sample delivers quicker results by reducing the need for a lengthy subculture step. Antibiotic susceptibility testing and identification testing via MALDI-TOF requires a clean bacteria sample. Background components from the blood culture such as cells, cellular debris, salts, proteins and others need to be removed in order to have reliable test results. In addition, the bacteria concentration in the output sample needs to be sufficiently high so that enough biomass is available (approximately 10⁸ CFU/mL) for ID by MALDI-TOF). The benefits of rapid identification (e.g., via MALDI-TOF) and antibiotic susceptibility testing (e.g. by microbiological or biochemical methods) are increased by quickly isolating the bacteria directly from a blood culture and avoiding the subculture step used widely today.

Tangential flow extraction can serve to isolate bacteria from a sample using the membrane device 100. For example, in one process, bacteria may be isolated from a positive blood culture. In an example of such a process, 10 mL patient blood sample is collected into a BACTEC bottle, and inoculated with bacteria such as *E*. *coli*, *S. aureus*, *S. pneumoniae*, or other bacteria. The sample is incubated in a BACTEC blood culture instrument until a positive blood culture is detected. Within 0-8 hours from detection, the positive blood culture is applied to a tangential flow extraction process where bacteria will be isolated and washed. Referring again to FIG. 6A, the membrane device 600 (or tangential flow extraction device), similar to the previously discussed examples, has a first chamber 610 (e.g., input or upper chamber) and a second chamber 620 (e.g., output or lower chamber) separated by a track etched membrane (TEM) 630.

The positive blood culture sample flows (repeatedly in a multi-pass process) along the track etched membrane in membrane device 600 in the first chamber (e.g., upper chamber; 104 in FIG. 1) and partial liquid phase flows through the track etched membrane 108 (TEM; FIG. 1) into the second chamber (e.g., lower chamber; 106 in FIG. 1) by applying a trans-membrane pressure.

The success of a tangential flow filtration using a membrane device 600 depends on several interrelated parameters of a filtration system selected for a target constituent. In this example, the target constituent is bacteria in positive blood culture. The critical parameters include membrane pore size, chamber geometry, tangential flow rate, and trans-membrane pressure. In addition, the sample to be processed may have variations in solid content, particle size distribution and concentration, viscosity, shear sensitivity, propensity to adhere to surfaces, degree of biofouling, coagulation etc. Highly efficient recovery of a range of potentially different bacteria from a large sample requires a balance of many parameters. Issues such as biofouling, membrane clogging, reduced viability of the target organism, low purity of the output sample, excessive processing time or low recovery may occur during the filtration process if the processing parameters and geometry are not chosen appropriately.

In single stage tangential flow filtration of blood culture where the target bacteria are to be retained, the membrane pore size is selected to prevent the bacteria (typically having a size of about 1 µm to about 2 um) from passing through the membrane pores. The pore size of the membrane is selected to be smaller than the typical size of the bacteria. However, if the membrane pore size is too small, it will result in a slow filtration process and potential retention of some undesired particles and debris in the retentate phase along with the bacteria. Membranes with very small pore sized require a larger trans-membrane pressure to drive the filtration since the flow resistance is typically higher as the pore size is reduced. If the membrane pore size is too large, the filtration time may be shorter but the target bacteria may pass through the membrane and the recovery rate will be reduced.

In tangential flow filtration, the tangential flow rate and trans-membrane pressure are normally chosen in order to provide a successful and efficient filtration. Desired efficiency or target efficiency means not only the percent recovery of the target bacteria and its purity are high, but also the filtration speed is optimized for the fixed filtration area. Higher tangential flow velocity generally helps prevent cake layer formation on the membrane surface or membrane clogging. The trans-membrane pressure is selected to provide a sufficient filtration rate but the trans-membrane pressure is not so large as to cause membrane clogging by pulling particulate matter into the membrane pores (reducing the effective diameter) or blocking the membrane pore entrances all together. This clogging occurs when the tangential flow velocity is too low even in devices having a low trans-membrane pressure. Membrane clogging can either significantly reduce the efficiency of the tangential filtration or make the filtration/purification process close to impossible. The membrane clogging problem is particularly severe when the solid content and/or viscosity of the sample is high as often seen in positive blood cultures.

In tangential flow filtration, the chamber geometry is an important factor which affects the filtration performance. There are typically two chambers in such devices, the chambers being separated by the membrane. The selection of chamber height, width and length affect the fluid flow resistance, which affects the tangential flow rate, velocity and effective trans-membrane pressure. For a fixed filtration area (width X length = constant), a higher chamber height will result in less flow resistance. In order to achieve enough flow velocity to prevent or minimize the cake layer formation on the membrane or membrane clogging, the flow rate has to be higher than the flow rate used for a low chamber height system. For most applications, a smaller chamber height provides for more efficient in filtration in each pass of the sample volume. The tangential flow resistance, however, is increased in this case, and a greater pressure differential is required to drive the flow along the membrane surface. Shorter chamber length and wider chamber width can reduce the flow resistance. Conversely, the introduction of an excessively wide uniform flow path at the sample inlet makes maintaining the flow velocity challenging. Hence, a proper corresponding geometry is required for the intended filtration parameters and performance for a specific input sample type and input volume.

In one embodiment, an example of the membrane device 600 can have a first chamber geometry of about 20 mm x about 42 mm x about 0.8 mm with a TEM membrane pore size ranging from about 0.2 µm to about 2.0 µm. More preferably, the TEM has a pore size in the range of about 0.4 µm to about 1 µm . Such pore size is selected to retain the bacteria in the retentate phase. Positive blood culture is introduced to a reservoir connected to the device inlet. The tangential flow is powered by a pump, syringe or pressure source etc. In this embodiment, the power source is a peristaltic pump which enables a recirculating flow allowing the sample to flow through the membrane device in multiple passes continuously in a setting similar to FIG. 5.

The tangential flow rate can be in a range of 10 to 90 mL/min and more preferably in a range of 40 to 70 mL/min. The trans-membrane pressure can be in the range of 0.5 (about 3.45 kPa) to 10 psi (about 68.95 kPa) and more preferably in the range of 3 (20.68 kPa) to about 8 psi (55.16). The trans-membrane pressure is applied by using a pressure source, vacuum, a pump, a syringe, or other conventional mechanisms. Tangential flow drives the sample solution along the membrane, while a pressure gradient across the filter/membrane creates the motive force to drive smaller constituents or particles smaller than the pore size of the membrane through the membrane. The volume of the retentate phase decreases and the bacteria becomes concentrated as the filtration process continues.

To reduce the number of constituents in the sample, the sample can be pre-treated to remove some constituents to reduce the chance of membrane fouling. For example, when the sample is a positive blood culture, the blood cells in the background of the positive blood culture are removed prior to the sample filtration process. This can be achieved by prefiltration. In another example, the blood cells in the background of the positive blood culture are selectively lysed prior to the sample filtration process. The selective lysis of the blood cells can be achieved by using a liquid lysing buffer or a dried down lysing agent which is dissolved or mixed with the positive blood culture. It should be noted that careful selection of the differential lysing agent is critical so that the blood cells will be lysed efficiently and quickly without affecting the viability of the bacteria or their response to the drugs in AST test. Such gentle lysis buffers are well known to the skilled person and not described in detail herein. Dissolved salts, proteins etc. in the background of a positive blood culture can be removed by rinsing or washing with additional washing buffer such as deionized (DI) water. This process can be repeated as necessary to purify the retentate phase to satisfy the bacteria purity requirement for MALDI-TOF testing. The bacteria retentate may have additional lysing and washing buffers added to further reduce contaminants that may adversely affect identification and antibiotic susceptibility testing results. In some implementations, an antifoaming agent is added to the lysing buffer and/or washing buffer to prevent foam formation during the filtration process, as many positive cultures can create foams during the filtration process depending on the bacteria type. Foam formation during the process can be detrimental to the process and the quality of the output sample. The antifoam agent is selected and used in quantities that will not affect the bacteria viability, MALDI-TOF ID or susceptibility to drugs in AST test. Anti-foam agents are well known to the skilled person and not described in detail herein.

To test the system described herein, positive blood cultures were processed using a single stage tangential flow filtration with 8 different bacteria species in three types of BACTEC media. The bacteria tested were *E*. *coli*, *S. aureus*, *S. pneumoniae, S. epidermidis, E. faecalis, K. pneumoniae, P. aeruginosa and E. cloacae.* Culture bottles used were a BACTEC standard bottle, a BACTEC plus bottle and a BACTEC lytic bottle. A blood sample (10mL) was collected into the respective BACTEC bottle, which was then inoculated with a bacteria species to simulate a bacteria concentration of ~50 CFU/mL in the blood volume. The sample was incubated in a BACTEC blood culture instrument until bacterial growth was detected. In BACTEC, growth is detected by monitoring changes in one of oxygen concentration, carbon dioxide concentration or a change in pH deemed indicative of microbial growth. The typical bacteria concentration in a positive culture ranges from about 10⁶ CFU/mL to 10⁸ CFU/mL. The positive blood culture (about 10 mL) is taken from the BACTEC bottle and lysed by mixing with 10 mL BACTEC lytic media (or other proper lysis buffer solution).

Traditionally, this mixing is performed manually by rotating by hand the container with the positive blood culture from an upright position to upside down position repeatedly. This mixing is sometimes also accomplished through additional features in the fluidic cartridge, such as a herring bone feature or bubbling the fluid. However, such a method is completely external to the fluidic path of the disposable cartridge. In one embodiment of the present invention, this mixing is automated and is accomplished by using a motor to rotate the disposable cartridge from an upright position to a rotation between 1-180°. Automation reduces hands on time for the technician, reducing labor costs.

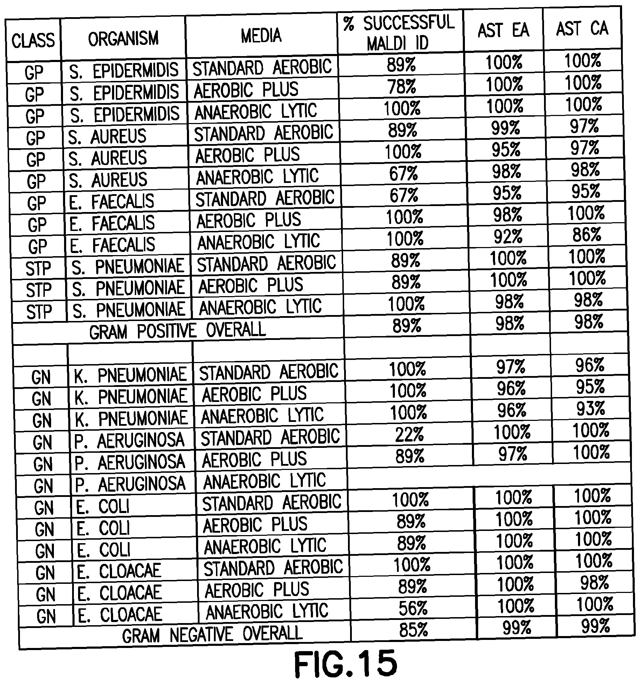

The 20 mL lysed mixture solution is input to a tangential flow extraction system as described herein. A filtration device similar to membrane device 600 as described above was used. The tangential flow chamber geometry on the retentate side (i.e. the top side as illustrate) of the membrane is 20 mm in width x 42 mm in length x 0.08 mm in depth with a membrane pore size of 0.8 um. The tangential flow rate was 60 mL/min. The flow of the sample through the filtration device was powered by a peristaltic pump at the outlet side of the tangential flow filtration chamber. The output of the peristaltic pump was fed back to the sample reservoir attached to the inlet of the membrane device in an arrangement similar to that illustrated in FIG. 5. Such an arrangement permits continuous recirculating tangential flow of the fluid sample multiple times over the same filtration membrane surface. A vacuum of 6 psi was applied to the permeate side of the chamber to provide a trans-membrane pressure that allowed the liquid phase of the sample containing dissolved components or smaller particulate matter to flow through the membrane. As the filtration process continues, the volume of the retentate phase is reduced. Bacteria is retained on the membrane and concentrated due to the fact that it is separated from the liquid sample constituents. When the retentate volume is reduced to about 2 mL, two consecutive washes with 2.5 mL deionized (DI) water are performed while the filtration process continues, followed by a secondary lysing step using 5 mL of the same lysing agent. The lysis reagent may already be present in the PBC, or added to the device. Reagents (e.g. the DI water, lysis reagent, etc.) can be prefilled on the device, added to the device manually or automatically via fluid connections from the device to reagent reservoirs. The filtration process continued and an additional four (4) consecutive DI water washes (2.5mL each) are used to further remove the undesired components. These was step remove the undesired sample components (e.g. salt, cell debris, hemoglobin etc.) from the target component (e.g. pathogens) and keeps the target components suspended in the fluid for recovery from the device after isolation, purification and or concentration in the device. After the final DI water wash, the filtration process is stopped when the retentate volume reaches approximately 1 to 1.5 mL and the isolated, purified and/or concentrated sample is collected for testing. Identification and antibiotic susceptibility resulting from a range of bacteria are illustrated in FIG. 15. In the data of FIG. 15, the MALDI results are based on an average of three biological replicates, with three technical replicates per biological replicate. The AST results are based on an average of three biological replicates. AST EA is the antibiotic susceptibility essential agreement and refers to the percent of conditions in which the minimum inhibitory concentration (MIC) is within one doubling dilution of the reference method MIC. AST CA is the antibiotic susceptibility testing categorical agreement and refers to the percent of conditions in which the susceptible, intermediate, or resistant interpretation agree with the reference method. For both AST EA and CA, the reference method uses was the Becton Dickinson Phoenix^{™} method according to the instructions on the product insert.

The above-described process can be modified to have more or less wash steps, more or less volume per wash, and more or less additional lysing steps depending on the requirements for processing time, sample purity, volumetric limitations, etc. Process parameters and/or geometry may be adjusted from those described above to suit a particular use of the method/device described herein.

### (3) Bacteria Recovery and Concentration from Urine Sample

As previously mentioned, the membrane device 100 can be implemented for a single stage extraction such as where the sample tangentially flows along one membrane filter. Such a process may be implemented for bacteria recovery and concentration from urine in a process similar to that described in relation to the prior blood sample example. The following example illustrates how tangential flow extraction can be applied to a urine sample for bacteria extraction. The urine samples were acquired and all were culture negative prior to receipt. The same device configuration, as used for fresh whole blood TEM concentration experiments previously mentioned, with a pore size membrane of about 0.2 µm to about 3 µm was implemented for the urine processing. In the test, urine samples were spiked with *E. coli* at a concentration of 56 CFU/10mL prior to the concentration process with a membrane device. A pressure control system (e.g. a system obtained from Fluigent) was used to provide 1 and 4 psi pressures on the input side (first chamber of the membrane device) to drive the multi-pass tangential flow. A 10 mL sample was thereby processed on a TEM device until the remaining volume reached 0.5 mL, which was recovered and plated on an EMB plate and cultured overnight. A total of five samples from separate subjects were used. In this preliminary test, the initial E coli concentration in the urine sample was 5.6 CFU/mL and the final *E. coli* concentration was 87 CFU/mL after the concentration process through the membrane device. The average recovery was 77% with a concentration factor of about 16-fold as shown in Table 2.

**Table 2: Average results for TEM device concentration for E. coli spiked urine samples, N=5**

| **Input Volume** | **Output Volume** | **Input *E. coli* Concentration** | **Output *E. coli* Concentration** | ***E. coli* Concentration Factor** | ***E. coli* Recovery** |
|---|---|---|---|---|---|
| 10 mL | 0.5 mL | 5.6 CFU/mL | 87 CFU/mL | 16X | 77% |

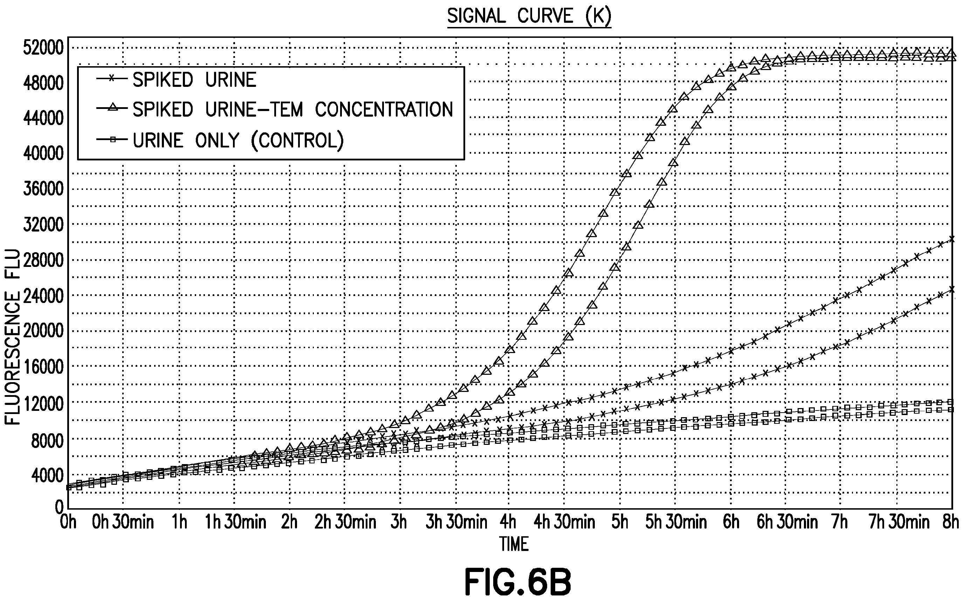

In another experiment, concentration of bacteria in urine sample showed a 10-fold increase in concentration of the bacteria from the original sample. β-lactamase (antibiotic resistance marker) was detected about 4 hours faster using TEM-implemented concentration of spiked urine samples than when using the unconcentrated urine specimen. Results are illustrated in the graph of FIG. 6B (top two curves illustrating the increase in speed).

### B. Multiple stage extraction process

As previously mentioned, the membrane device 100 can be implemented for a multiple stage extraction such as where the sample components tangentially flow along several membranes such as where the membranes of each stage have different pore characteristics (e.g., size). Such a process may be implemented for bacteria recovery and concentration or even exosome extraction and concentration from fresh or diluted whole blood.

### (1) Two stage exosomes extraction and concentration

Exosomes are extracellular vesicles that range in particle size from 120 nm to 30 nm found in blood or urine samples. They are secreted through the endosome multi-vesicular body complex by most cells and convey information to neighboring or remote cells by delivering RNA and proteins thus affecting various physiological and pathological signaling pathways. Exosomes have been widely investigated due to their availability through a non-invasive liquid biopsy process and their potential diagnostic value such as for cancer detection. Extraction of exosomes has been done by several techniques including the gold standard process using ultra centrifugation, precipitation and affinity-based or size exclusion column technologies. These technologies can be time consuming, tedious procedures or suffer from poor quality or low recovery efficiency.

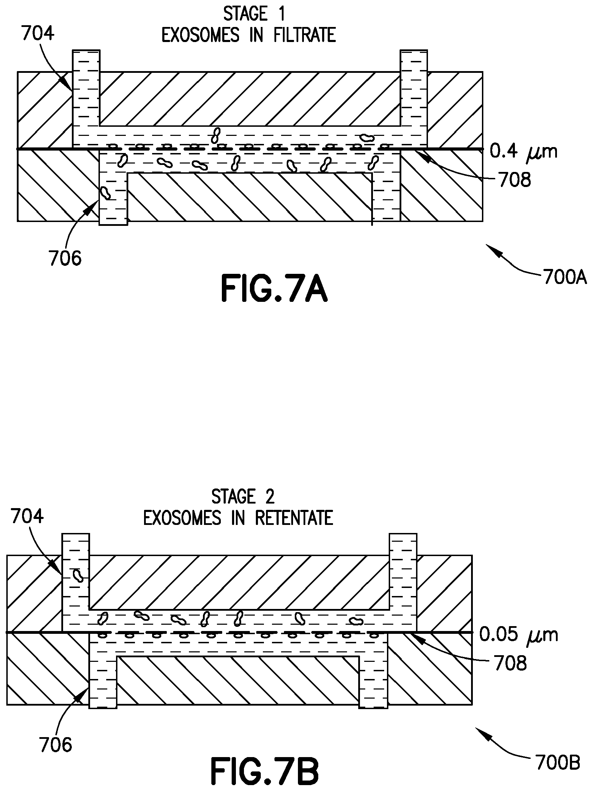

The present technology permits a new process for extracting exosomes from biological samples such as using a multiple stage tangential flow extraction with membrane devices. Such a process is described with reference to FIGs. 7A and 7B. For the first stage process of FIG. 7A, whole blood is collected from a patient; large particles such as red blood cells (RBCs), white blood cells (WBCs) and platelets are isolated from the blood. As illustrated, this process can be achieved by tangential flow extraction to separate plasma from the larger blood components using a membrane device as previously described. Alternatively, such extraction/separation can be achieved instead with normal centrifugation. In relation to the tangential flow process illustrated, exosomes remain in the plasma phase along with other species such as proteins, peptides and dissolved salts. Such plasma separation from the blood can be achieved using a membrane device such as a TEM with pore size on the order of about 0.2 µm to about 1 µm (0.4 µm as illustrated in FIG. 7A). The TEM may be sandwiched between a first chamber (chamber 704) for tangential flow and a second chamber (chamber 706) for collecting plasma with exosomes. A trans-membrane pressure is applied to drive the plasma flow through membrane 708 (e.g., TEM) of the membrane device 700A (output side of the membrane at chamber 706) while keeping the cells and platelet in the first chamber (chamber 704) (input side of the membrane at chamber 704).

The second stage as illustrated in FIG. 7B may be implemented with a further membrane device 700B to process the output (i.e. the permeate) of the membrane device 700A. The device will process the plasma containing exosomes at the sample input (first side of membrane 708 at chamber 704) and isolate the exosome particles at the input side by removing the dissolved species such as proteins, peptides, DNA etc. to the output (second side of membrane 708 at chamber 706). The extraction can be achieved by a TEM tangential flow process using a device consisting of a first chamber (chamber 704) and a second filtrate chamber (chamber 706) separated by a membrane 708 (e.g., TEM). The membrane pore size can be on the order of 0.1 µm or smaller (0.05 µm as illustrated). The plasma sample flows tangentially along the track etched membrane in the first chamber and liquid with dissolved salts and other smaller particles flows through the membrane by applying a trans-membrane pressure. The engineered system allows a balanced dual flow along (tangential) and through (trans) the TEM (membrane 708) where exosomes are retained at the first chamber (chamber 704) side of the membrane and are enriched. Additional process such as washing the exosomes by introducing a washing buffer to the first chamber at this stage and repeating the process can further purify the isolated exosomes. Alternatively, a washing buffer can be added at the beginning of the second stage or in the middle of the second stage of exosomes extraction. The washing process can be repeated if necessary.

Alternatively, additional stages can be added to the process in order to extract exosomes of different sizes and/or to increase the purity of the extracted exosomes. For example, after the first stage of plasma separation using 0.4 µm pore TEM and the second stage of 0.05 µm pore TEM process as described above, the sample (e.g., retentate from stage 2) can be further filtered using a membrane device with a membrane having a pore size on the order of 0.1 or 0.2 µm to remove larger particles. This third filtration stage can also be performed between the first stage and the second stage process.

In an example of the second stage filtration of FIG. 7B using a tangential flow TEM device with 0.05 µm pore size, the plasma filtration rate was characterized and confirmed to be feasible. Table 3 below shows that the filtration rate varies with the trans-membrane pressure.

**Table 3**

| Vacuum (kPa) n=3 | Input Vol. (µL) | Average Retentate Vol. (µL) | Average Filtrate Vol. (µL) | Average TEM Filtration Rate (µL/min) |
|---|---|---|---|---|
| 20.68 (3psi) | 1000 | 298 | 536 | 16.11 |
| 41.37 (6psi) | 1000 | 258 | 573 | 24.46 |
| 62.05 (9psi) | 1000 | 253 | 584 | 28.60 |

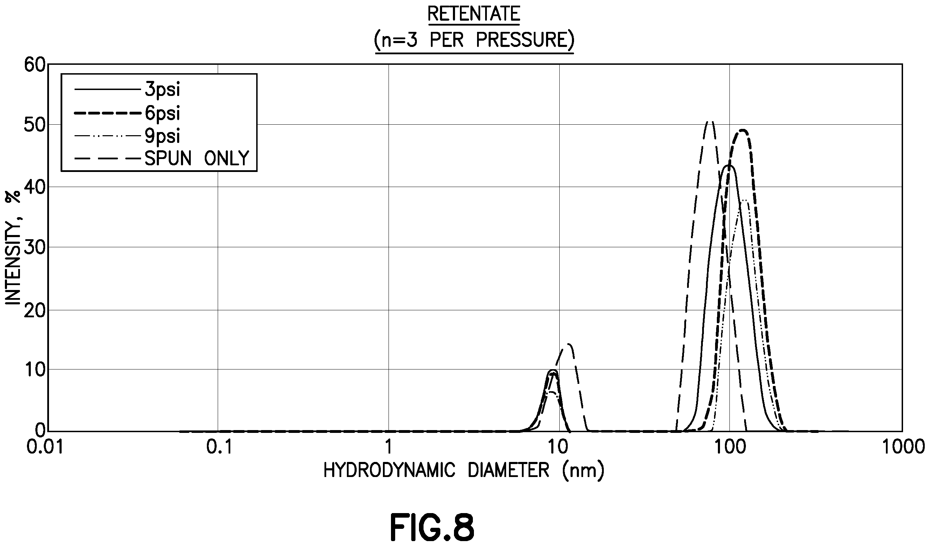

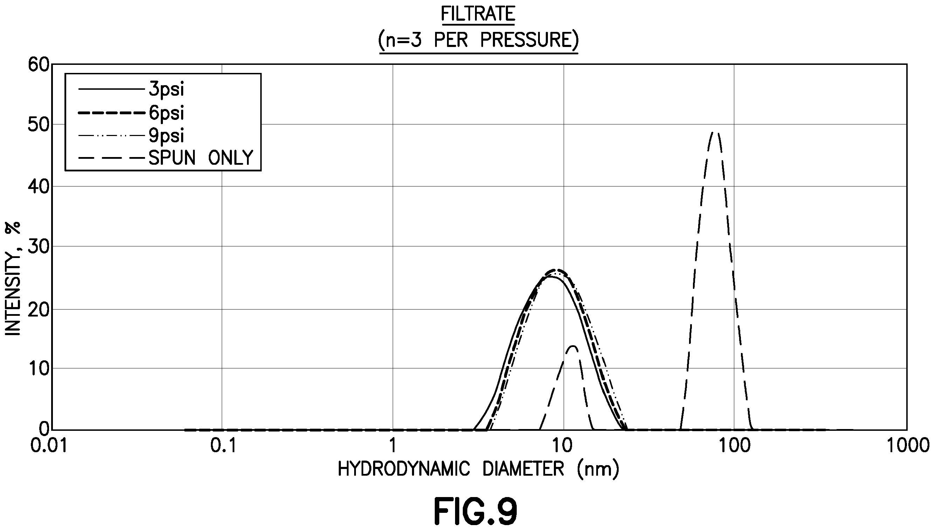

Particle size analysis using dynamic light scattering (DLS) showed that the hydrodynamic radius of the extracted particles matches the expected size range of exosomes. The particle size analysis as a function of trans-membrane pressure is illustrated in FIGs. 8 and 9. The filtrate after the 0.05 µm pore size filtration showed average particle size around 10 nm for all three transmembrane pressures 20.68 kPa (3 psi), 41.37 kPa (6 psi) and 62.05 kPa (9 psi). No particles with a hydrodynamic radius more than 50 nm were found. The retentate contains particle sizes around 100 nm on average, which are typical exosome particle sizes. As illustrated in FIG. 9, the transmembrane pressure did have some influence on the average particle size collected in the retentate. FIG. 8 (retentate) and FIG. 9 (filtrate) illustrate the intensity-weighted hydrodynamic sizing of the extracted exosome particles using a two-stage process as described, without the washing process.

### (2) Two stage bacteria extraction from whole blood, diluted blood or a positive blood culture

When extracting bacteria from whole blood where a lot of red blood cells are present, a multiple stage process (at least two, or more) can be used to achieve the purity and concentration level of the target substance (e.g. bacteria). For example, a two-stage process can be implemented where the first stage process will remove large cellular particles (for example, red blood cells, white blood cells, or platelets) from the whole blood, diluted whole blood or whole blood culture sample and the larger debris (in case of lysed blood). The larger debris are particles greater than a predetermined pore size that will allow the bacteria or other target substance(s) to pass through the membrane. The bacteria remain in the permeate phase that passes through the membrane in the first stage process. An illustration of the tangential flow and trans-membrane (filter) flow allowing bacteria to pass through the membrane is illustrated in FIG. 13.

In order to allow the bacteria to pass through the membrane device, a membrane with pore sizes on the order of the size of the bacteria or larger should be used. It was found that a track etched membrane with pore size on the order of about 2 to about 5 µm are suitable for this purpose. As described earlier, the membrane device includes a first chamber (an input chamber) and a second chamber (an output chamber) separated by a membrane (e.g., TEM). A trans-membrane pressure is applied to drive the bacteria through the membrane (i.e. into the permeate) while keeping the cells and platelets in the first chamber (i.e., the retentate). Both tangential flow and trans-membrane flow (as previously described) can be powered by pressure and/or vacuum sources at the input side and/or output side. The tangential flow can be one-directional flow. One directional flow can be a single-pass tangential flow or a multi-pass rotational flow in the first chamber such as a re-circulating flow powered by a fluid pump.

Alternatively, the tangential flow may be reciprocating flow where blood flow reverses its direction to allow fluid flow over the same membrane surface multiple times to drive the filtration process to completion at limited sample input volume and to reduce membrane fouling.

The second stage process may take the bacteria rich permeate from the first stage as the sample input and be processed using a secondary membrane such as a further TEM for tangential flow in another membrane device. This secondary TEM tangential flow process using a device having a first or upper chamber and a second or lower chamber separated by a TEM of pore size of about 0.2 µm to about 1.2 µm. As the bacteria rich permeate from the stage 1 filtration process is used as the input sample to second stage, and the sample flows along the track etched membrane in the upper chamber, liquid flows through the membrane by applying a trans-membrane pressure. The engineered system allows balanced dual flows along (tangential) and through (trans) the TEM membrane where bacteria are enriched, retained and remain viable in the first chamber. The tangential and trans flow rates are selected to prevent fouling of the membrane due to phenomena such as cake layer formation or pore clogging with particulate and to optimize the sample processing time without introducing undesired shear or lysing of cellular components. The bacteria remaining in the first or upper chamber can be recovered for next step testing such as detection, identification or antimicrobial susceptible test. Optionally, additional washing can be used to further purify the sample by removing dissolved salt, proteins, nucleic acids, peptide, lipids etc. such as using one or more additional membrane devices. The washing buffer or other suitable liquid can be introduced at the beginning, during or at the end of concentration process. The concentration process can be repeated after addition of buffer solution in order to reach a targeted concentration factor.

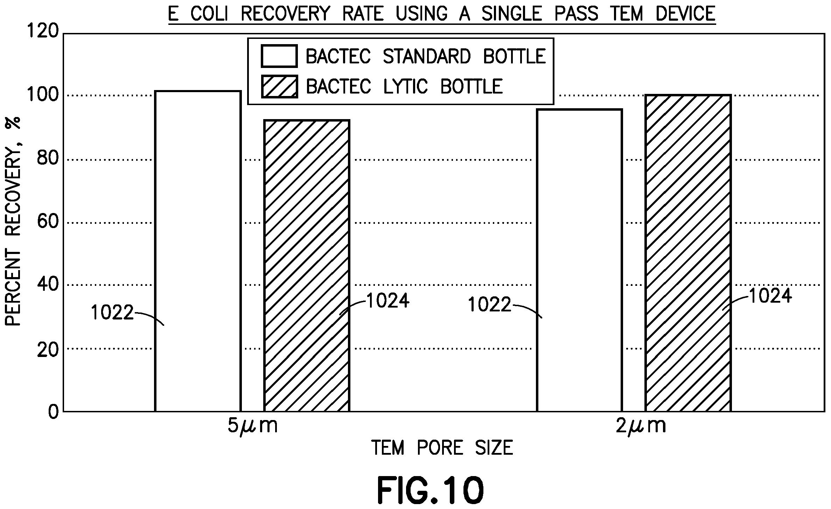

In an example of such a process, the performance of a larger pore size TEM device for a first stage in a multistage process was evaluated with bacteria in whole blood in BD BACTEC lytic (lysed blood) and BD BACTEC standard bottles (not lysed blood) although other types of blood culture media could be used. The initial screening used track etched membranes with pore sizes of about 2 µm and about 5 µm respectively. It was found that *E. coli* in the blood can pass through membranes with either a 2 µm and 5 µm pore size with high efficiency (>95%). In the graph of FIG. 10, bacteria recovery rate is plotted showing that both lytic bottle (bar 1024) and standard bottle (bar 1022) had good bacteria recovery.

A TEM device with a membrane pore size of 2 about µm was selected to conduct another test to understand the efficiency of separating blood cells from bacteria from the positive blood culture sample. A bacteria-rich blood sample was created by using *E. coli* spiked blood in a BACTEC Standard bottle with incubation. A single pass TEM device was used to separate the bacteria from the positive blood culture. A 5 mL bacteria-rich blood sample was processed by a single pass process powered by a vacuum source. Vacuum power was varied to study the effect of trans-membrane pressure on bacteria recovery and blood cell removal after the filtration process. The recovered bacterial solution was further analyzed to determine the residue complete blood count (CBC), plasma free hemoglobin and *E. coli* concentration. in order to estimate the purity of bacterial suspension. Results in Table 4 below showed that permeate yield, bacteria recovery rate, and residual CBC in the permeate phase do not change much as the trans-membrane pressure (TMP) changes. The only residue in the plasma/bacteria phase is a small portion of platelets. This is an indication that the process has a relatively large operating window. It should be noted that a multi-pass process was also conducted and it has similar separation performance as in a single pass process.

**Table 4**

| **Device #** | **TMP, PSI** | **Yield (mL)** | **WBC (× 10³/µL)** | **RBC (× 10⁶/µL)** | **Platelet (× 10³/µL)** | **CFU × 10⁷/mL** | **E Coli, % Recovery** |
|---|---|---|---|---|---|---|---|
| Control 0 | - | N/A | 2.6 (1) | 0.912 | 26.4 | 116 | N/A |
| Device 1 | 2.5 | .92 | <LOD* | <LOD | 6.0 | 108 | 93 |
| Device 2 | 3 | 1.01 | <LOD | <LOD | 5.0 | 110 | 95 |
| Device 3 | 3.5 | 1.01 | <LOD | <LOD | 7.0 | 105 | 91 |
| Device 4 | 4 | 1.07 | <LOD | <LOD | 6.3 | 116 | 100 |
| Device 5 | 4.5 | 1.18 | <LOD | <LOD | 6.3 | 120 | 103** |

| | | | | | | | |
|---|---|---|---|---|---|---|---|
| *Below low detection limit ** Within experimental error | | | | | | | |

From the above, it is clear that the permeate contains very little white blood cells and red blood cells, since the amount is below the limits of detection (LOD). The yield in the permeate was slightly higher for the devices with higher transmembrane pressure, with slightly higher recovery of *E. Coli.* This preliminary test results showed that the separation of bacteria from whole blood is possible.

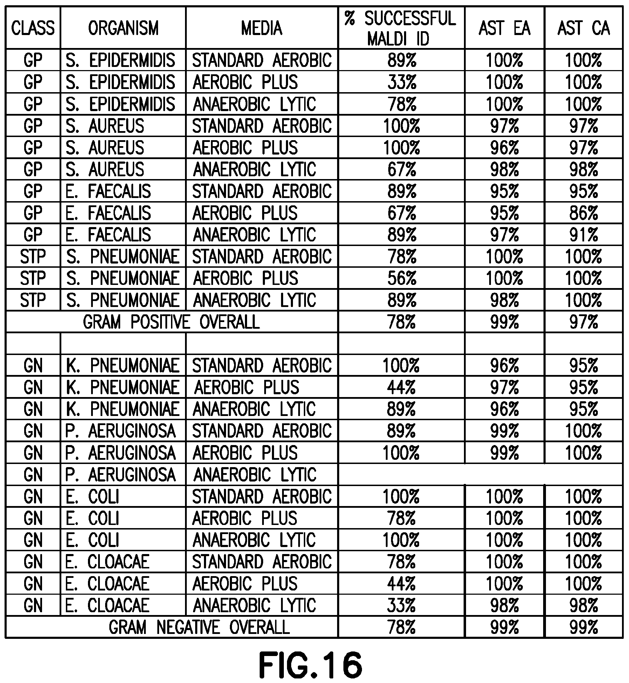

As described above, tangential flow extraction can serve as a concentration and washing process that will rapidly isolate bacteria from positive blood cultures (either whole blood samples or diluted blood samples) for identification and/or antibiotic susceptibility testing. In an example of such a process, about 8 mL to about 10 mL of patient blood sample is collected into a BACTEC bottle, and inoculated with bacteria such as *E. coli*, *S. aureus*, *S. pneumoniae,* and other bacteria. The sample is incubated in a BACTEC blood culture instrument until a positive blood culture is detected. Within about 8 hours or less from detection, the positive blood culture is applied to a tangential flow extraction process where bacteria will be isolated and washed. The membrane device 600 (or tangential flow extraction device), similar to the previously discussed examples, has a first chamber 610 (e.g., input or upper chamber) and a second chamber 620 (e.g., output or lower chamber) separated by a track etched membrane (TEM) 630. The sample flows (repeatedly if a multi-pass process is being used) along the track etched membrane in membrane device 600 in the first chamber (e.g., upper chamber (104 in FIG. 1) and partial liquid phase flows through the track etched membrane 108 (TEM; FIG. 1) into the second chamber (e.g., lower chamber; 106 in FIG. 1) by applying a trans-membrane pressure. This first stage of the filtration process may have a pore size ranging from about 2 µm to about 5.0 µm. Such pore size is selected to enable the bacteria to pass through into the permeate phase. Within a brief period of time (e.g., about 0.5 to about 5 minutes), 20 mL of the PBC sample is processed, with viable bacteria in the permeate phase and host blood cells and debris in the retentate phase. The stage 1 permeate (about 17 mL) is then processed by the second stage. The second stage of the filtration process may have a pore size ranging from about 0.2 µm to about 1.2 µm. Such pore size is selected to enable the bacterial to remain in the retentate phase. The permeate from stage 1 may have additional lysing or washing buffers added to further reduce contaminants which may adversely affect identification and antibiotic susceptibility testing results. Tangential flow drives the feed solution (e.g., the permeate from stage 1) along the membrane, which can prevent fouling as discussed in more detail herein, while a pressure gradient across the filter/membrane creates the motive force to drive smaller constituents or particles smaller than the pore size of the membrane, through the membrane. Within a brief period of time (e.g., about 10 to about 20 minutes) bacteria is concentrated as volume is reduced as required to enable the required concentration level with the necessary purity. Identification and antibiotic susceptibility results from a range of bacteria processed by this two-stage filtration process are illustrated in FIG. 16. In the data of FIG. 16, the MALDI results are based on an average of three biological replicates, with three technical replicates per biological replicate. The AST results are based on an average of three biological replicates. AST EA is the antibiotic susceptibility essential agreement and refers to the percent of conditions in which the minimum inhibitory concentration (MIC) is within one doubling dilution of the reference method MIC. AST CA is the antibiotic susceptibility testing categorical agreement and refers to the percent of conditions in which the susceptible, intermediate, or resistant interpretation agree with the reference method. For both AST EA and CA, the reference method uses was the Becton Dickinson Phoenix^{™}method utilizing an 18-24 subculture step.

### (C) Further Embodiments

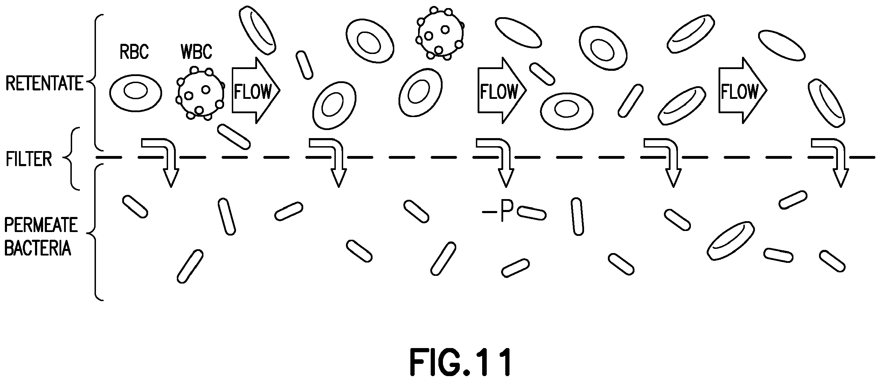

In another example, the tangential flow membrane device technology may be implemented to provide a bandpass filter solution(s) employing a two-stage process that separates/extracts constituents of interest, such as pathogens of interest, from both larger and smaller components in the sample based on inherent size differences of the various components/constituents. The filter passband, such as the one illustrated in FIG. 11, retains large extraneous components (e.g., blood cells) while the specimen of interest (bacteria) and small debris pass through to the retentate. Such a filtration process may be considered in relation to the following stages:
1. High-pass Filter membrane device. One stage of filtration employs a membrane device as a high-pass filter that passes smaller components of a sample, such as a bacterium of interest, while excluding larger components, such as red and white blood cells. The high-pass filter membrane device employs a membrane (e.g., TEM) with pore size(s) in a range sufficiently larger than about 0.5 µm and less than about 8 µm such as in a range from about 2 µm to about 5 µm in diameter to establish a cutoff size that excludes blood components that are typically larger than about 5 µm in diameter.
2. Low-pass Filter membrane device. In another stage of filtration, a membrane device is employed as a low pass filter. For example, the bacteria and accompanying debris that passed through the high-pass filter membrane device is then subsequently processed with a membrane device serving as a low-pass filter. The low-pass filter uses a membrane such as a TEM with chosen diameter pore size(s) to retain the relatively large bacteria and allow debris, analytes and waste to be removed. For example, such a pore size may be on the order of about 0.4 µm to about 1 µm for the low-pass filter membrane.

The end result is the sample of interest separated from the blood background, in a rapid fashion (e.g., on the order of about 5 to about 20 minutes), and ready for downstream processes, such as identification testing, resistance marker testing, susceptibility testing, plating, measuring turbidity, culturing, sequencing, and/or other processes that are routinely performed in a microbiology, clinical, or research laboratory.

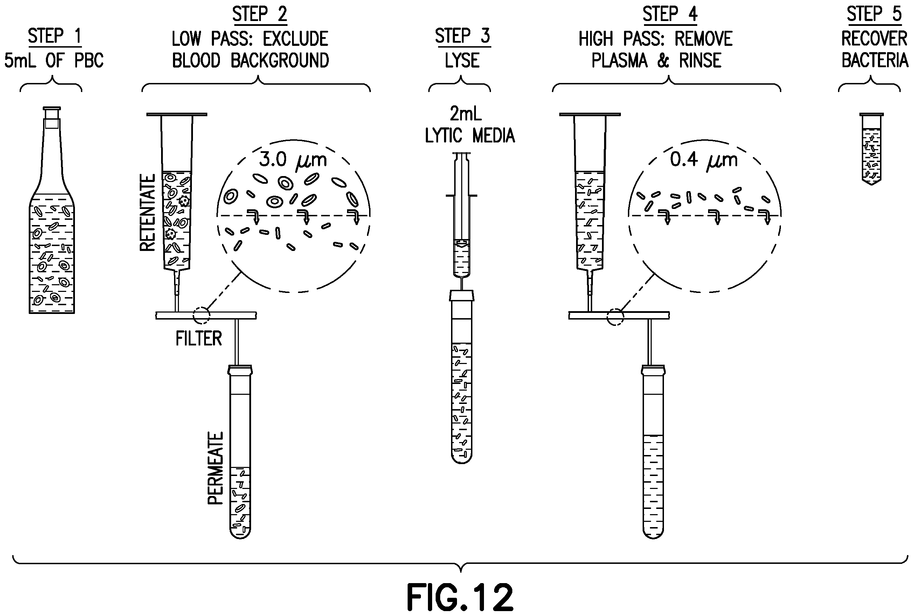

Such an example process is further illustrated in the process flow chart of FIG. 12. In step 1, a blood culture is obtained. In step 2, the blood culture is applied to a tangential flow membrane device which produces a permeate of sample constituents smaller than the filter size, such as 3.0 µm. Optionally, in lyse step 3, the permeate may be combined with a Lytic media. The permeate may then, in step 4, be applied to another tangential flow membrane device which produces another permeate of constituents smaller than the filter size, such as 0.4 µm, removing smaller constituents from the retentate of interest. At step 5, the bacteria are recovered from the concentrate of the retentate of step 4. Example apparatus for such a process is illustrated, for example, in FIG. 13.

### (D) Technology Benefits

Such technological processes as described in this specification may have various benefits. For example, the technology could facilitate identification and antimicrobial susceptibility testing directly from a positive blood culture (PBC) without the need for further subcultures, reducing time to results by as much as approximately eighteen (18) hours or more. This reduced time to results can have significant positive impact on clinical therapeutic decisions, resulting in improved patient outcomes, as well as institutional cost savings.

Current methods for processing a PBC include traditional subculturing or centrifugation techniques. Previous attempts are severely limited by processing times and complicated procedures.

Alternatively, traditional centrifugation techniques may be employed to isolate bacteria directly from the PBC to overcome this delay, but they require a number of laborious centrifugation and washing steps to obtain a pure sample ready for downstream ID/AST tests.

The proposed technology may overcome the limitations of such processes by providing a solution that isolates the sample of interest from the PBC in a short period of time, such as less than about 15 to 30 minutes, without the need for multiple centrifugation or washing steps, both decreasing the time to results and the number of laborious procedures. Further, the present technology can be readily automated, unlike centrifugation approaches, to enable high throughput processing.

Previous technological attempts to directly process a PBC using filtration traditionally relied on dead-end filtration techniques. In dead-end filtration, the feed solution is only driven perpendicular to the membrane and the resulting pressure is used to force particles smaller than the pore diameter across the filter. The particles larger than the pore diameter, however, build up on the surface of the membrane to form a cake that eventually fouls the filter. Common solutions to overcome this limitation is to lyse and digest the PBC sample prior to filtration in an attempt to reduce the overall size of particles to restrict the cake from forming. Previous studies have shown though that lysing and digesting the sample has a negative impact on the viability of the target microorganisms that impacts the performance of downstream ID/ AST studies, effectively precluding prior art attempts from adoption.

The proposed technology as described herein can rely on tangential flow filtration that offers significant advantages over dead-end filtration such as to prevent filtration clogging. Tangential flow filtration (as described in detail herein) continuously washes the filter as the solution is fed tangentially along the membrane to prevent fouling, such as with a peristaltic pump in a multi-pass operation. It also can provide a benefit of repeated filtration of a given sample so as to improve concentration while avoiding clogging. As a result, the PBC input (as is, lysed or digested) can be more flexible for this tangential flow filtration, further reducing complexity and increasing performance.

As discussed herein, the apparatus with bandpass filter membrane devices may be enabled with tangential flow filtration (a crossflow filtration). As described, tangential flow filtration (TFF) may be understood to be a type of filtration that passes the feed solution (the sample) tangentially along the surface of the membrane rather than merely directly perpendicular as in dead-end filtration. A significant advantage of TFF is that the tangentially flowing feed solution reduces caking the membrane and fouling the membrane pores, effectively increasing both performance and duration of operation. Repeated flow of the solution over the same surface area reduces the opportunity for loss of targeted microorganisms to the membrane surface, since effective filtration can be achieved by multiple passes of the sample over the same surface. Additionally, since the sample is exposed preferentially to the membrane top surface as opposed to a large interior surface area (as with fibrous membranes), there is reduced opportunity for loss of target microorganisms due to adherence to the membrane compared to approaches where the sample is allowed to pass through a thickness of fibrous or another higher interior surface filter. Additionally, a pressure differential is formed normal to the membrane that serves as a motive force to drive particles smaller than the diameter of the pores across the membrane, such as by application of a negative pressure differential (e.g., a constant negative pressure differential) held with a vacuum or vacuum pump.

The examples of the disclosed technology can isolate bacteria of interest from the PBC (or other biological sample) by separating sample components based on inherent differences in size using, for example, a bandpass approach. The technology can employ multi-pass tangential flow filtration as described herein.

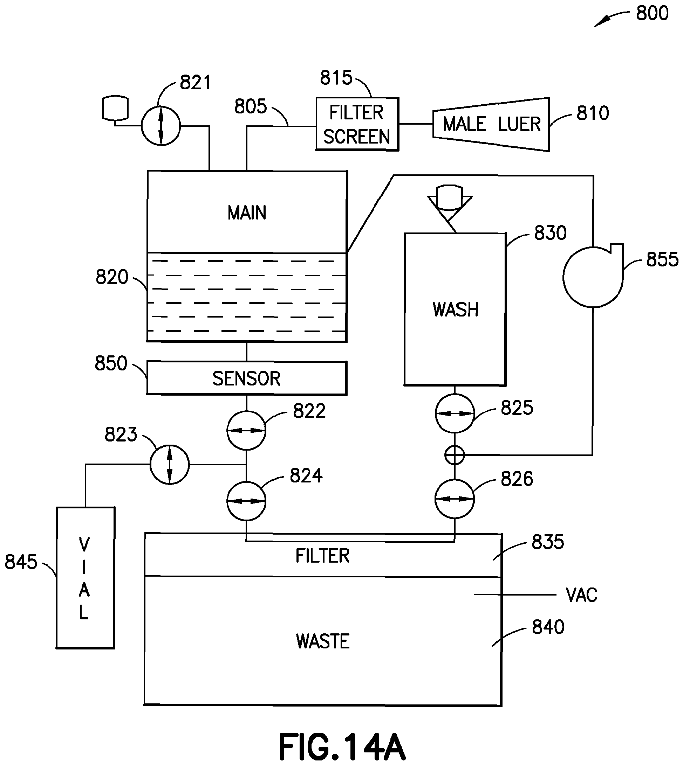

Optionally, a disposable cartridge is used to isolate bacteria from the other parts of the sample using a tangential flow membrane device. The operation of one example of such a disposable cartridge is described in FIG. 14A-K. As illustrated, the cartridge 800, which is formed on a cartridge substrate (not illustrated), has a fluid pathway 805 for drawing sample from a syringe or culture bottle (not illustrated) into the cartridge and subsequently processing the sample. The sample is drawn from a syringe or culture bottle through port 810. One example of a suitable port 810 is a luer connector.

The sample is drawn through a filter screen or porous membrane 815. The pore size of the filter or porous membrane is selected to prevent resin in the syringe or culture bottle from flowing into main chamber 820. The pore size for a filter or membrane that will selectively prevent the resin from passing through the membrane or filter, yet allow other sample constituents to pass through, is well known to one skilled in the art. Membrane pore sizes in the range of about 25 µm to about 100µm are contemplated, but the skilled person may select another pore size if so warranted by the resin particle size. Resin particles are typically at least about 10 µm or more in diameter, which is much larger than the other sample constituents (red blood cells, white blood cells, microbial contamination (if present) etc.

Although illustrated as a seemingly planar arrangement, in one embodiment the port 810, the filter 815 and the vial 845 are on one side of a cartridge substrate (not shown) and the main compartment 820, filter 835, wash chamber 830 and other components for process the sample and illustrated in FIGs. 14A-14K are on the opposite side of the substrate.

Referring to FIG. 14A, the device 800 is in its initial state with all of valves 821, 822, 823, 824, 825 and 826 closed. When the cartridge is in this state, main chamber 820 is prefilled with lysis buffer and wash chamber 830 is filled with wash buffer. Lysis buffers and wash buffers that are suitably mild to ensure that microbial contamination isolated from the biological sample remain viable for downstream analysis are well known to the skilled person and are not described in detail herein.

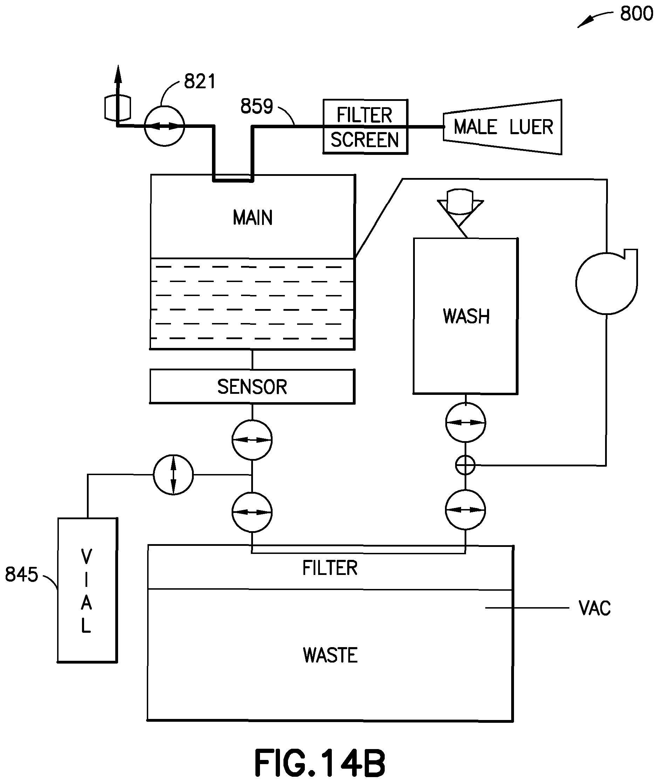

Referring to FIG. 14B, after the device 800 is installed in the instrument (not shown), valve 821 is opened to vent the fluid pathway 805. This is illustrated by flow path 859. The sample bottle (not shown) is connected to the port and the valve 821 is then closed again.

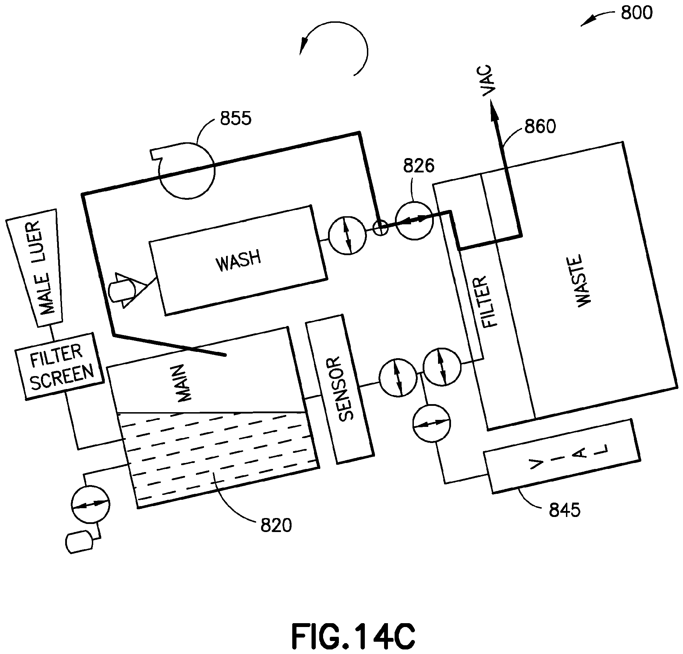

In FIGs. 14C-F, the device 800 is rotated from the upright position (e.g., FIG. 14B) to the side position (e.g., FIG. 14C). The sample processing requires the PBC culture bottle to be upright for certain step (i.e. venting) and with neck facing slightly downward for other steps (aliquoting) .

Referring to FIG. 14C, the device 800 is rotated to the position in which the sample (i.e., the positive blood culture (PBC)) will be drawn into the device. In this position the filter output valve 826 is opened and a vacuum is drawn through line 860. The pump 855 (e.g., a peristaltic pump) is run to pull a vacuum in the main chamber 820. The main chamber 820 is exhausted to the atmosphere through the vacuum manifold as illustrated by flow path 860. In some embodiments, a volume of about 2 ml-20 ml can be automatically aliquoted from the positive blood culture. Currently, manual techniques are used to aliquot volumes less than 2 ml and to vent PBC bottles. Venting is typically done by placing a needle (not shown) at atmospheric pressure into the septum of the PBC bottle. Traditional aliquoting means utilize a syringe with a needle, or a vacutainer connected to a needle system to withdraw sample from culture bottle. Both methods work for small volumes, but encounter significant difficulties when drawing larger volumes due to lack of headspace in the bottle. The neck of the PBC bottle is long and narrow, so a normal needle length will not reach the fluid level when the bottle is upright. To overcome this, technicians often turn the bottle upside down to shift the fluid into the neck of the bottle. Although this method works for most media types, bottles such as the BACTEC^{™} Plus bottles contain resin beads that fill and settle in the neck of the bottle. If a technician tries to use a normal syringe in that position, the resin beads will clog the tip of the needle preventing fluid flow. If the technician rotates the bottle at an angle rather than upside down, they are able to draw 1-2 ml volumes from the bottle but still with difficulty. The resin beads can continue to fully or partially clog the needle.

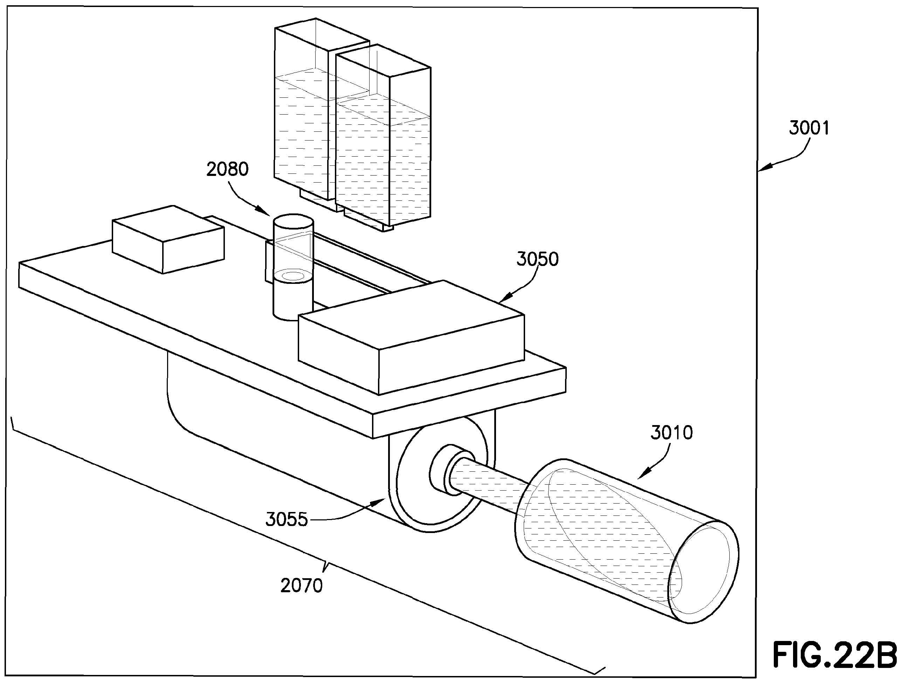

The cartridge described herein solves these issues and facilitates an advantageous work flow. In the first step, the PBC bottle is attached to a cartridge. This is illustrated with reference to FIG. 22B. There, the bottle 3010 can be seen as inserted into a port 3055. In one embodiment the PBC bottle 3010 enters the cartridge 2070 in an upright position. This port 3055 contains a material (such as a sponge) that contains a disinfectant. This is to ensure the surface of the PBC bottle septum does not contaminate the PBC sample. As the PBC bottle 3010 is pressed into the disinfectant material, a needle (not shown) punctures the septum of the PBC bottle 3010. The instrument then actuates a valve (not shown) and vents the PBC bottle 3010 (e.g. through a hydrophobic vent). To reduce any risk of aerosolization, hydrophobic vents with pore sizes (for example, about 0.1-0.45 µm) are provided to prevent microbial contamination (e.g. bacteria) from escaping the system, yet allow the release of vacuum or pressure from the PBC bottle 3010.

Referring back to FIG. 14C, once the PBC bottle is rotated, it is advantageous to pause for between about 1-120 seconds to allow the resin beads to settle at the base of the bottle. An instrument can be used to rotate the PBC bottle 3010 (FIG. 22B)/disposable device 800 from an upright position to an angle where the neck of the bottle is equal to or below parallel (e.g., 0°-25°) with a small motor (not shown). To aliquot the PBC bottle (3010 in FIG. 22B), the instrument utilizes an instrument pumping mechanism 855 (for example, vacuum, peristaltic, etc.) to draw about 2-20ml of the contents of the PBC bottle 3010 (FIG. 22B) into the main fill chamber 820 of the disposable cartridge 2070. All fluids remain in the device 800 and have no direct contact with the instrument. The draw volume is defined by the main fill chamber 820 geometry in combination with an automatic fill sensor 865 that detects when the chamber is filled and stops the automated fill. As the fluid fills the main fill chamber 820 in the disposable device, the fluid eventually fills an area in front of a capacitive sensor of the automatic fill sensor 850. Once the capacitive sensor detects the fluid, it stops the sample draw and the instrument rotates the PBC bottle 3010 (FIG. 22B)/device 800 back to the upright position. In this embodiment, the volume of the main fill chamber 820 defines the volume of the aliquot drawn from the sample bottle 3010 (FIG. 22B). To prevent any resin beads from entering the disposable device 800, a membrane is disposed within a housing (not shown). The membrane filters the beads either by lateral or through flow as the sample from the PBC bottle 3010 (FIG. 22B) is drawn into the device 800 (FIG. 14C)

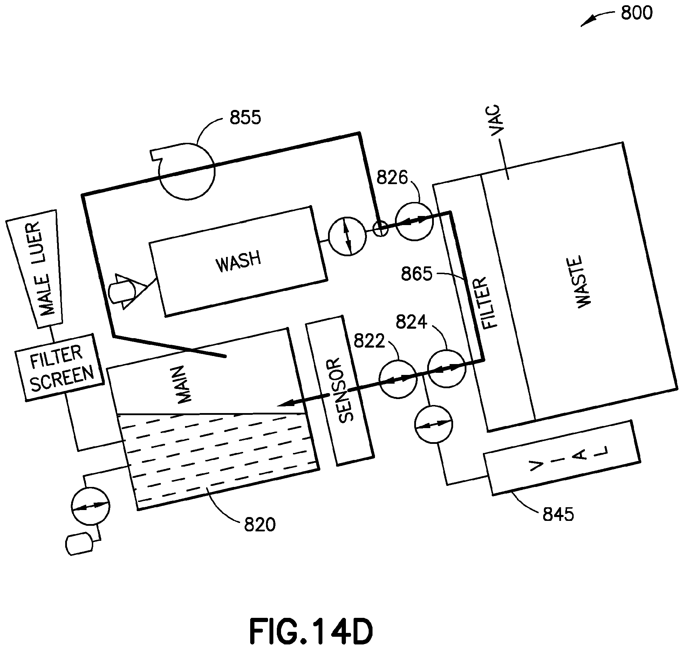

Referring to FIG. 14D, the device 800, in line valves 822 and 824 are opened and lysis buffer in the sensor channel 865 is purged into the main chamber 820. The peristaltic pump 855 is then turned off and the filter output valve 826 (illustrated as open) is then closed.

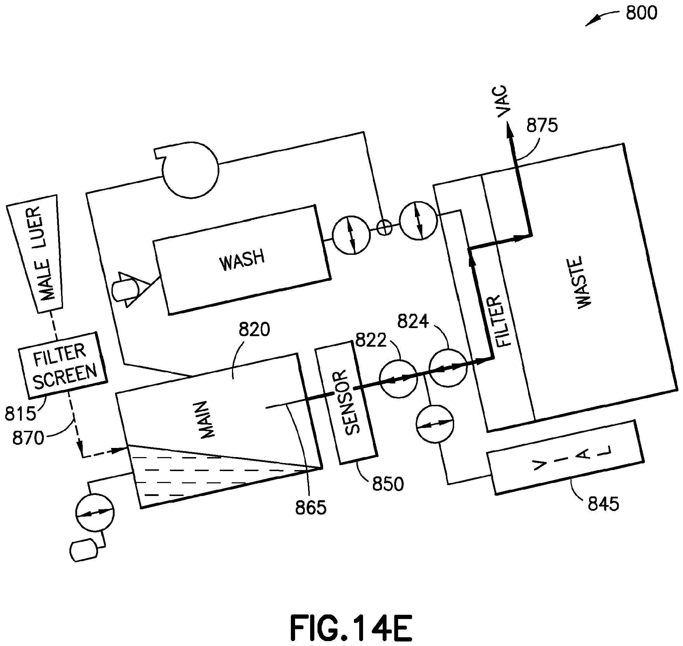

Referring to FIG. 14E, the device 800 is then operated to draw the PBC into the main chamber 820 through flow path 870. As noted above, membrane 815 prevents resin in the PBC from entering main chamber 820. However, the remainder of the PBC sample passes through membrane 815 to enter the chamber through flow path 870. Valves 822 and 824 are opened and the vacuum 875 is on to draw the PBC sample into the main chamber 820. Once the PBC fills main chamber 820 sufficiently, the PBC will be drawn into sensor channel 865. The sensor 850 will then turn off the vacuum 875 and close valves 822 and 824 (illustrated as opened).

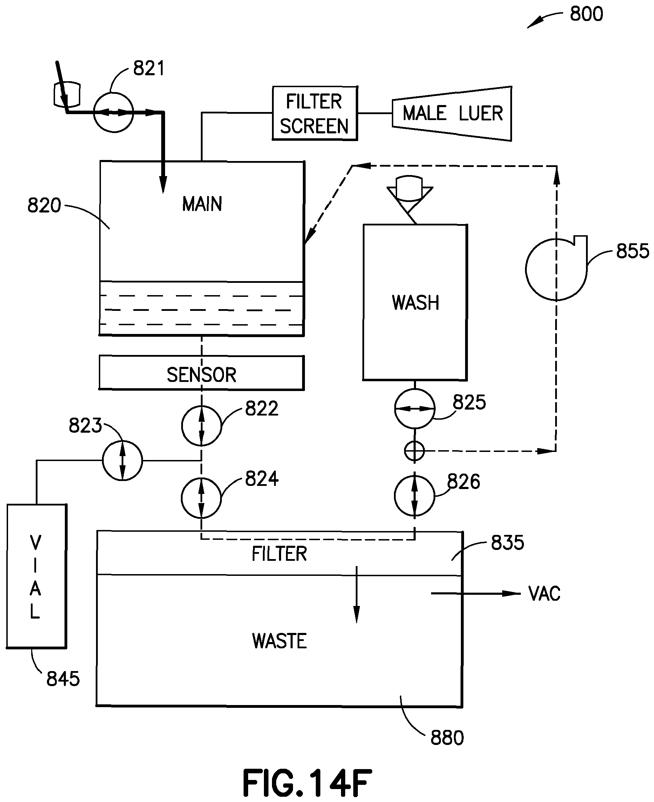

Referring to FIG. 14F, the device 800 is then rotated to an upright position. Valves 821, 822, 824 and 826 are opened, causing air to flow into main chamber 820 through valve 821. The PBC flows from main chamber 820 through filter 835 drawn by vacuum 875. The permeate from the sample flows through the filter membrane 835 into the waste chamber 880 as described herein. The retentate, carrying the microorganisms, flows tangentially along the filter membrane 835. The peristaltic pump 855 moves the retentate fluid back into the main chamber 820.

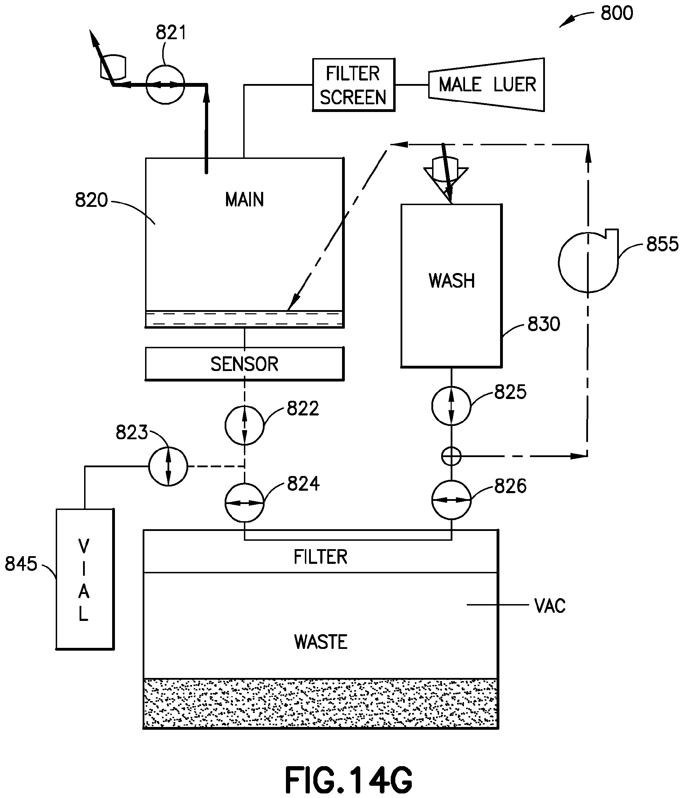

Referring to FIG. 14G, valves 824 and 826 are closed and the wash chamber valve 825 is opened to permit the wash buffer to flow from the wash buffer chamber 830 into the main chamber 820. The main chamber 820 is vented to the atmosphere in this configuration. The peristaltic pump 855 is activated for a predetermined time to meter in a predetermined amount of wash buffer into main chamber 820. After the wash buffer is added to the main chamber 820, the device 800 resumes the configuration described in FIG. 14F to further filter the contents of the main chamber 820. The operation of the device is controlled through a predetermined number of filter and wash cycles. When the system reaches the final wash cycle, the peristaltic pump 855 is activated for a longer set time to ensure the wash chamber is completely empty. The wash chamber is emptied so that the device can be purged of retentate and the retentate extracted as described below.

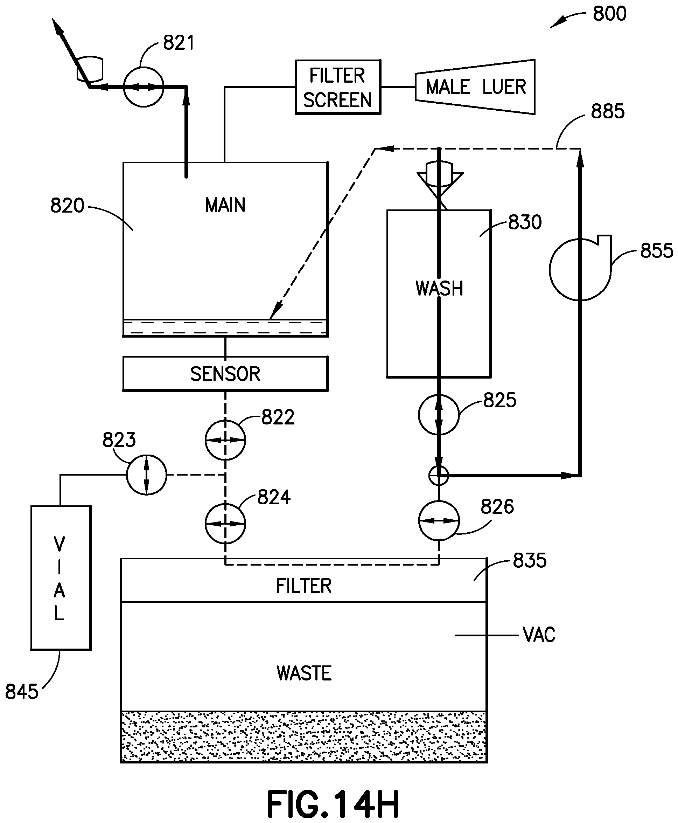

Referring to FIG. 14H, in the first purge stage, the device 800 purges the retentate from the retentate flow loop described above back into the main chamber 820. In the first purge stage, peristaltic pump 855 is activated to clear the return channel 885 that is between the exit of the filter 835 and the main chamber 820. As described above, the wash chamber 830 is empty at this stage and valve 825 is opened and the channel is vented, thereby purging the channel 885.

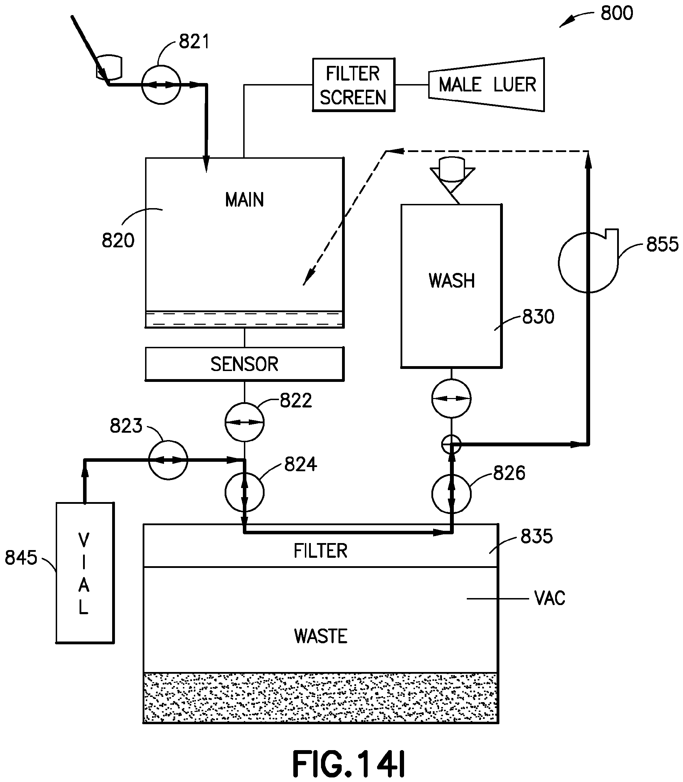

Referring to FIG. 14I, in the second purge stage, the peristaltic pump 855 is activated to clear the filter 835 and evacuate air out of the extraction vial 845. In this state, valves 821, 823, 824 and 826 are opened. Under this condition, a vacuum is created inside the extraction vial 845. The vial 845 may be evacuated in other stages of the process. In one embodiment, the vial is under vacuum when assembled with the system. In another embodiment, the vial can be evacuated through vacuum line. In that configuration valves 823 and 824 are open and at least valves 822 and 826 are closed.

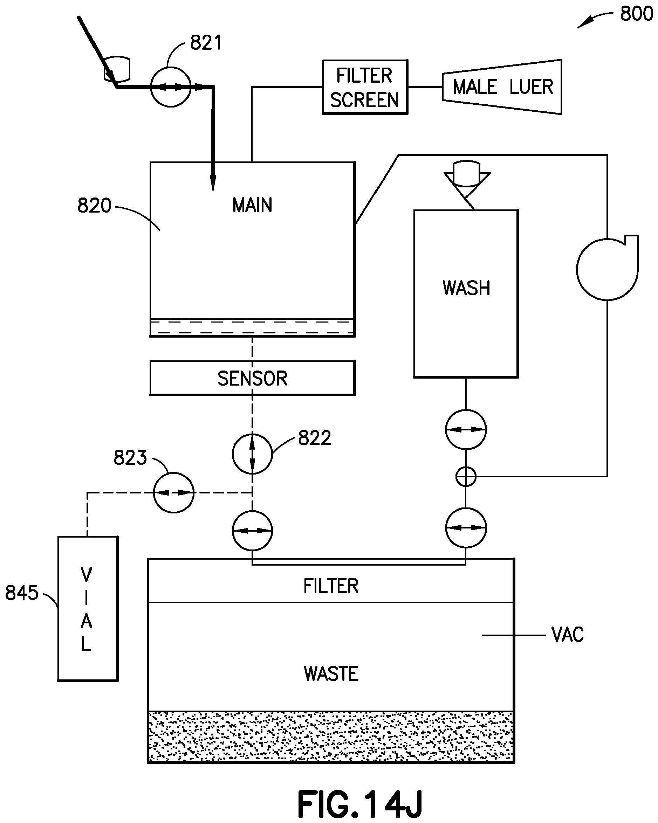

Referring to FIG. 14J, the peristaltic pump 855 is turned off and valves 821, 822 and 823 are opened, and all other valves are closed. The flow path is from the main chamber 820 to the vial 845. Because the vial 845 is under vacuum, the retentate in the main chamber 820 is drawn into the vial.845

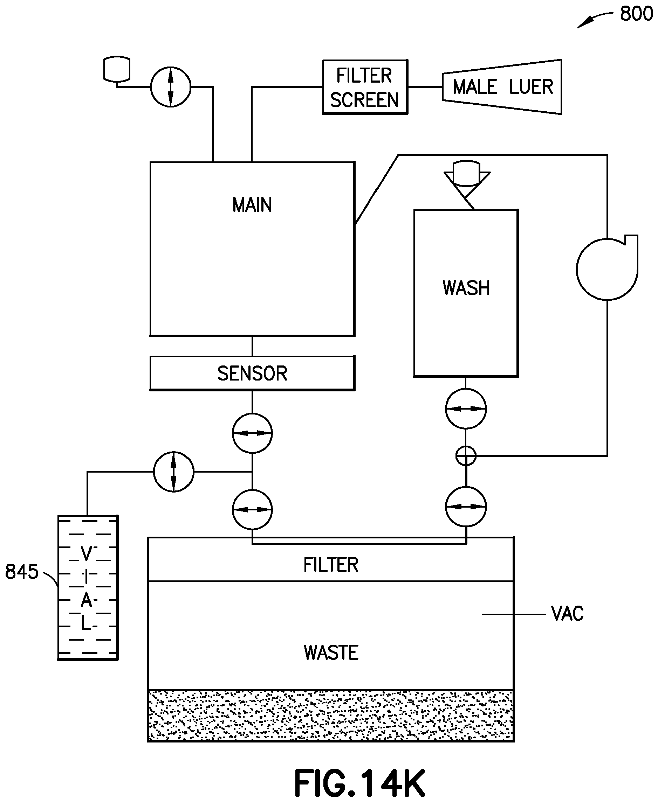

Referring to FIG. 14K, all of the valves in the device 800 are closed. The vial 845 is detached from the device and the retentate therein is used for microbial testing (or other downstream uses). The remaining portions of device 800 can be discarded . It is noted in this regard that the peristaltic pump, the vial and the sample bottle are attached to the device, but are not a part of consumable device. The device has tubing that is coupled to the peristaltic pump. However, the sample does not pass through the pump itself, allowing the pump to be reused even though the consumable device is not.

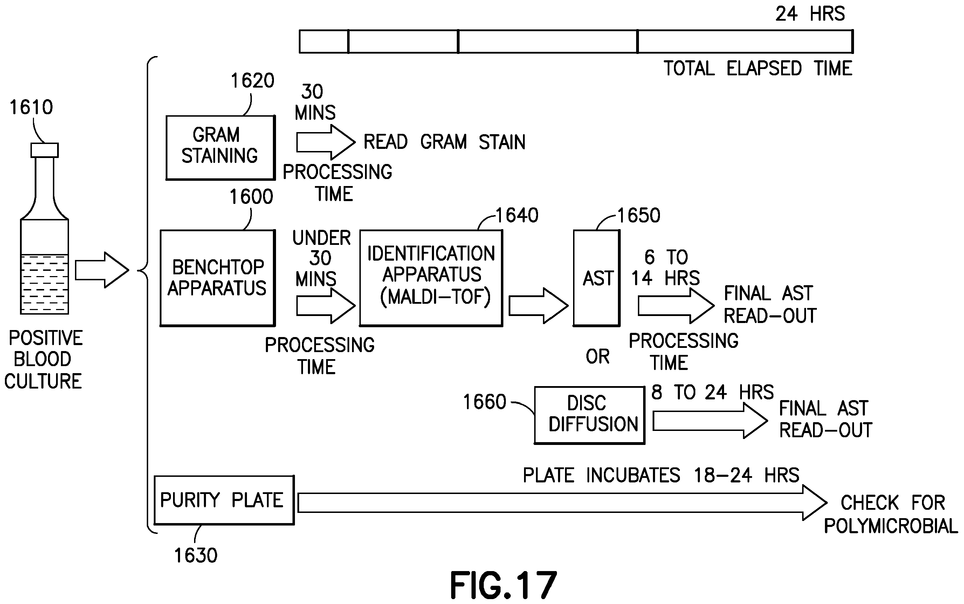

FIG. 17 illustrates the 30-minute processing time of a benchtop apparatus 1600 adapted to receive disposable cartridges that carry either the single stage or multiple stage tangential flow membrane devices described previously. FIG. 17 also illustrates how the benchtop apparatus 1600 can be integrated with conventional workflows for ID and AST sample assessment. The benchtop apparatus replaces the need to subculture pathogens recovered from a positive blood culture. As noted above, only about 30 minutes is needed to obtain the component of interest from the sample (e.g. the PBC) 1610 that is the source for sample in the single stage or multiple stage membrane device. After 30 minutes, the isolated, purified, and/or concentrated component of interest (e.g. pathogens) can be delivered to an identification apparatus (e.g. MALDI-TOF) 1640 or an apparatus for antibiotic susceptibility testing (AST) 1650. The results of the MALDI-TOF can be obtained instantly. AST results take approximately 6 to 14 hours. If AST is performed using disc diffusion 1660, results can take from 8 to 24 hours. If a subculture is performed prior to AST, the AST result will take about 18 additional hours. The benchtop apparatus avoids the need to prepare a subculture. The benchtop apparatus can be used in parallel with gram staining 1620 or a purity plate 1630 to identify polymicrobial infections.

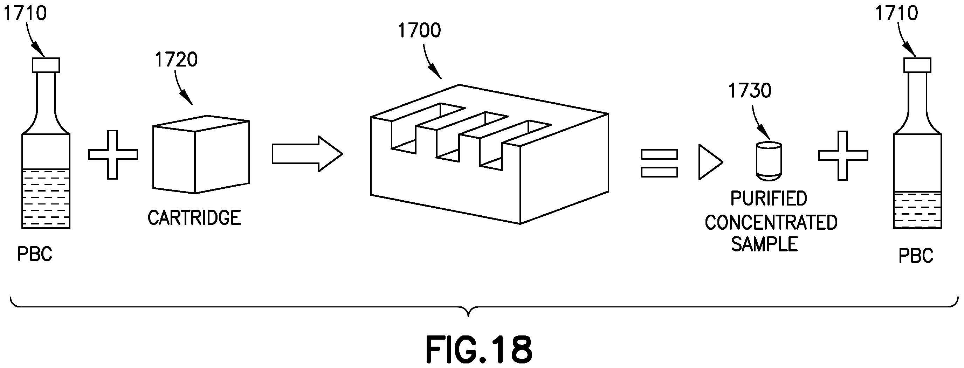

FIG. 18 illustrates the basic components of a benchtop apparatus 1700 that can receive sample such as from a positive blood culture bottle 1710. The benchtop apparatus 1700 is configured to receive one or more cartridges 1720 that can contain either the single stage or the multistage tangential flow membrane device described herein. The cartridge 1720 includes a vial 1730 that can receive the isolated, purified and or concentrated component of interest (e.g. pathogens) from the single stage or the multistage tangential flow membrane device. The vial 1730 can be separated from the cartridge 1720. The cartridge 1720 can be removed from the instrument 1700 and is then discarded. In one example, the user inputs the PBC bottle 1710 or aliquot from the PBC bottle 1710 into the cartridge 1720 that is then placed in the benchtop apparatus 1700. The benchtop apparatus 1700 automatically processes the sample to output a suspension of the target sample component (e.g. bacteria). If the target component is bacteria, that isolated, purified and/or concentrated sample is ready for ID and/or AST testing. As noted above, the time from PBC to the start of ID and/or AST testing is only about 30 minutes. The size of the instrument is a matter of design choice, but typical benchtop sizes (e.g. about the size of a microwave) are contemplated. Alternatively, the benchtop instrument can be integrated into an automated workflow such as BD Kiestra^{™} TLA (total lab automation system). The benchtop apparatus 1700 is fully compatible with current ID and AST instrumentation and methods, since the instrument outputs the sample for use in those instruments and requires no modifications to such instruments. The benchtop apparatus 1700 is illustrated as capable of receiving three cartridges 1720, but a benchtop apparatus instrument can be configured to receive more or fewer cartridges. The system does not need to be monitored and can be integrated with automated or manual process flow for preparing samples for ID and AST testing.

Currently, the vials 1730 that are commercially available contain a screw cap and require manual sterilization of the cap before attaching to the disposable cartridge 1720, and before the user accesses the vial 1730 with a syringe. Current bottles 1710 would require a secondary method for attaching the vial 1730 to the disposable cartridge 1720 to maintain positioning during processing. In some embodiments of the present invention, the vial 1730 securely connects to the cartridge 1720, thus preventing user exposure, and allowing the user to access the contents of the vial 1730 once under a biosafety cabinet via a syringe or pipette. In one embodiment of the present invention, the cap of the vial 1730 has several features, including the following illustrative features, which may be present independently of one another: (1) The cap can contain a sponge or other material that contains a disinfectant. This ensures any small amount of liquid that escapes during detachment is trapped in the sponge and sterilized. (2) The cap can also contain a built-in septum, such as a rubber septum, that a needle can easily pierce and will reseal after removal. The septum facilitates the release of the vial 1730 from the cartridge 1720 without risk of spilling or leaking. (3) The cap can also be threaded such that it can be unscrewed from the vial 1730 once in a biosafety cabinet. This gives the user several options for accessing the highly concentrated microbial sample, with safety measure to reduce the risk of the user. (4) The cap can contain a mechanical notch that locks the output vial 1730 into the disposable cartridge 1720 to ensure the vial 1730 does not dislodge from the cartridge 1720 during processing. (5) The system 1700 may pull a vacuum on the output vial 1730. This vial 1730 can then drive fluid flow into the vial 1730 as a final step of the processing.

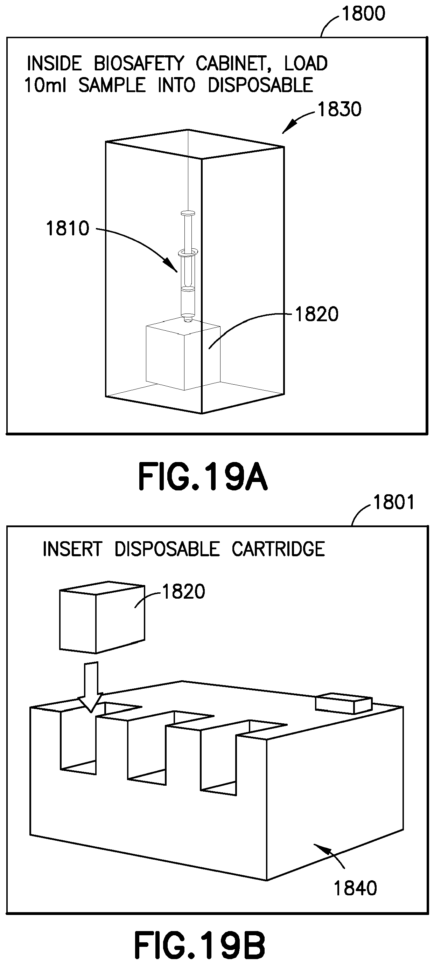

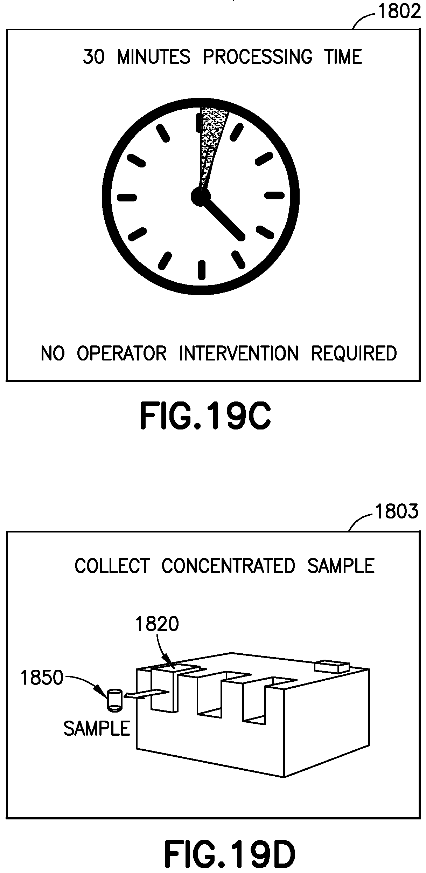

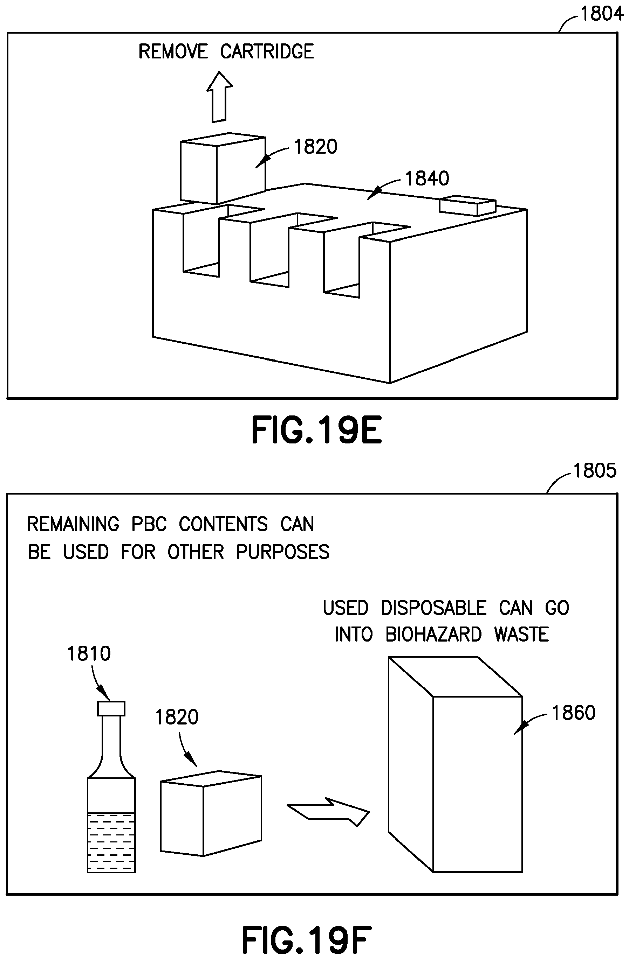

FIGs. 19A-F illustrate a workflow for the instrument used to process a PBC to isolate, purify and or concentrate pathogens in the PBC. In step 1800 (FIG. 19A), the cartridge 1820 that contains the tangential flow membrane device described herein is placed in a biosafety cabinet 1830. The cartridge 1820 is inoculated with the PBC from device 1810. In step 1801 (FIG. 19B), the cartridge 1820 is inserted in the benchtop apparatus 1840. The cartridge 1820 contains a tangential flow membrane device such as those illustrated in FIGs. 5 and 13 herein. The PBC is processed completely in the cartridge 1820 and does not enter the benchtop apparatus 1840. The benchtop apparatus 1840 can provide the necessary pressure to move the PBC through the tangential flow membrane device, without receiving the PBC from the device. In step 1802 (FIG. 19C), the PBC is circulated and recirculated through the tangential flow membrane device in cartridge 1820. This step takes about 30 minutes. In step 1803 (FIG. 19D), the isolated, purified and/or concentrated component of interest (e.g. pathogens) are collected into vial 1850 which is separated from the cartridge 1820. In step 1804 (FIG. 19E), the cartridge 1820 is removed from the benchtop apparatus 1840. In step 1805 (FIG. 19F), it is noted that only 10 to 20 mL of the PBC was required for the method and that a portion of the PBC remains for additional testing or discarded into biohazard waste. The cartridge 1820 is also disposable and discarded.

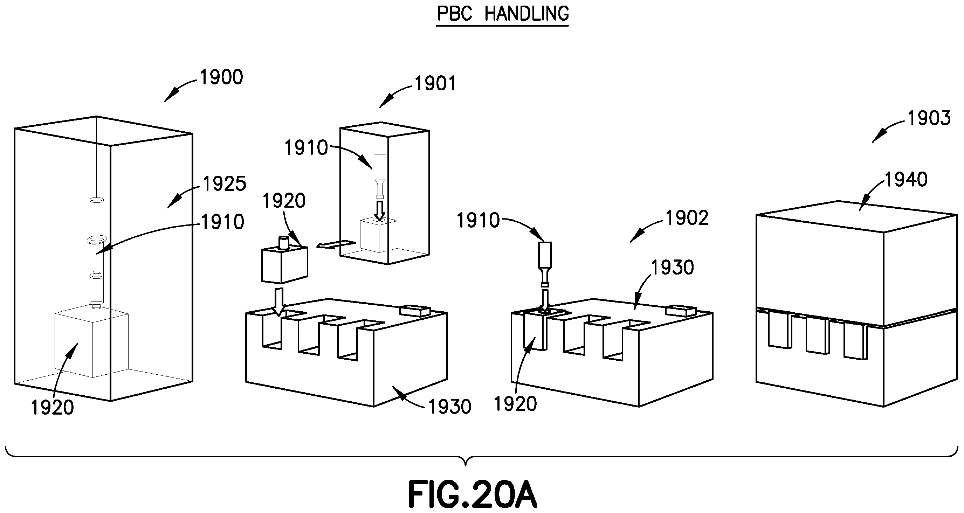

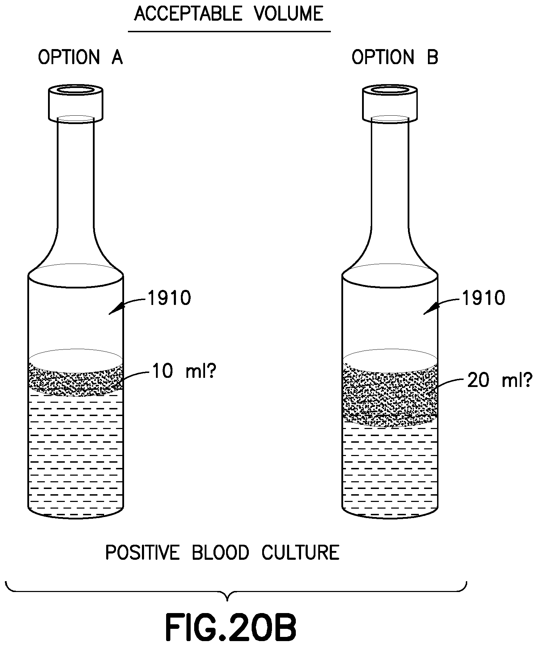

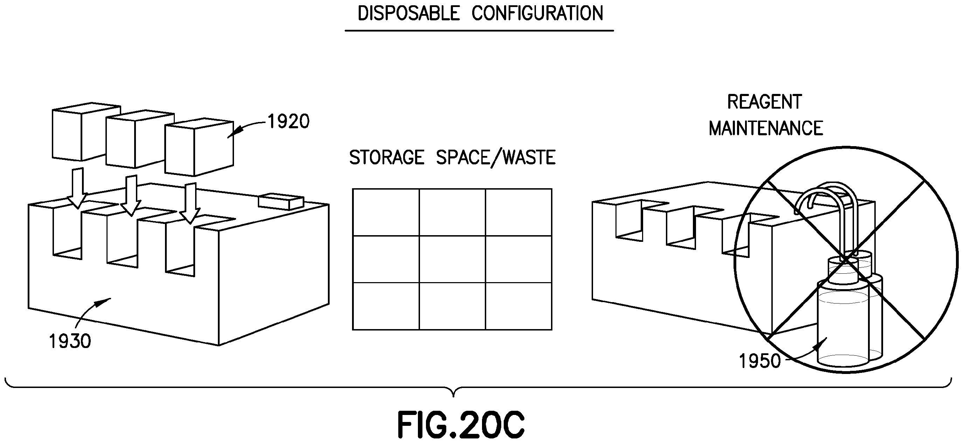

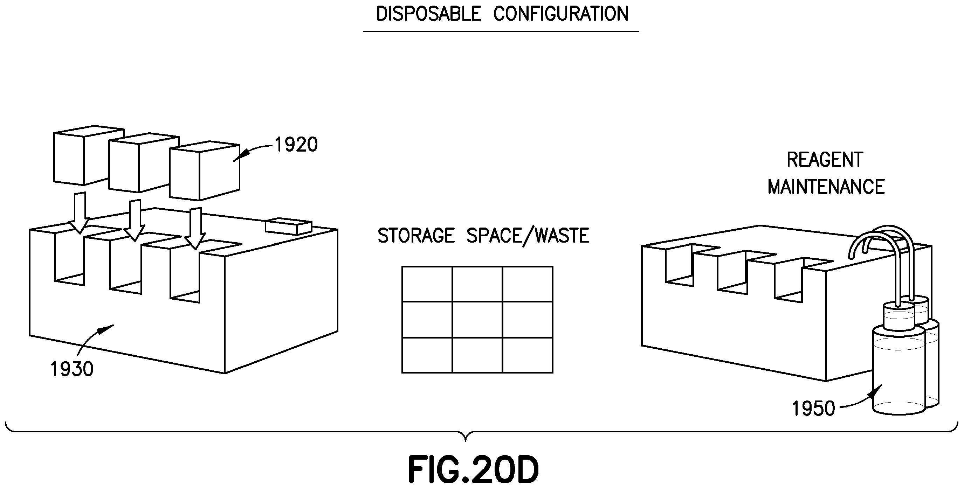

FIGs. 20A-D illustrate different ways in which the sample is loaded into the cartridge. In 1900 of FIG. 20A, the PBC container 1910 is inoculated into the cartridge 1920 in a safety cabinet as described above. In one option, the sample from the PBC is dispensed into a disposable while in a biosafety cabinet before going into the system. In 1901 of FIG. 20A, the PBC container 1910 is inserted into the cartridge 1920 while in the biosafety cabinet 1925. The cartridge 1920 then carries the PBC container 1910 into the benchtop apparatus 1930. In 1902 of FIG. 20A, the PBC container 1910 is inserted into the cartridge 1920 that is already held in the benchtop apparatus 1930. There is no fluid contact between the sample and the benchtop instrument 1930. In 1903 of FIG. 20A, an integrated system 1940 has the blood culture container in communication with the tangential flow membrane device. In this configuration, there is no need to manually place the PBC container 1910 into contact with the cartridge 1920, since such transfer occurs automatically. FIG. 20B observes that a portion of the PBC (e.g. 10 mL or 20 mL) is processed through the tangential flow membrane device, leaving a portion of the PBC for other uses. FIGs. 20C-D also illustrate that the reagents 1950 (e.g., the washing buffer or the lysis buffer described above) used in the tangential flow membrane device can either be provided pre-packaged in the cartridge 1920 (FIG. 20C) or integrated with the instrument 1930 (FIG. 20D). Reagent integration with the benchtop apparatus 1930 will require fluid connections with the tangential flow membrane device and therefore does not provide complete isolation of the chamber contents from the benchtop apparatus.

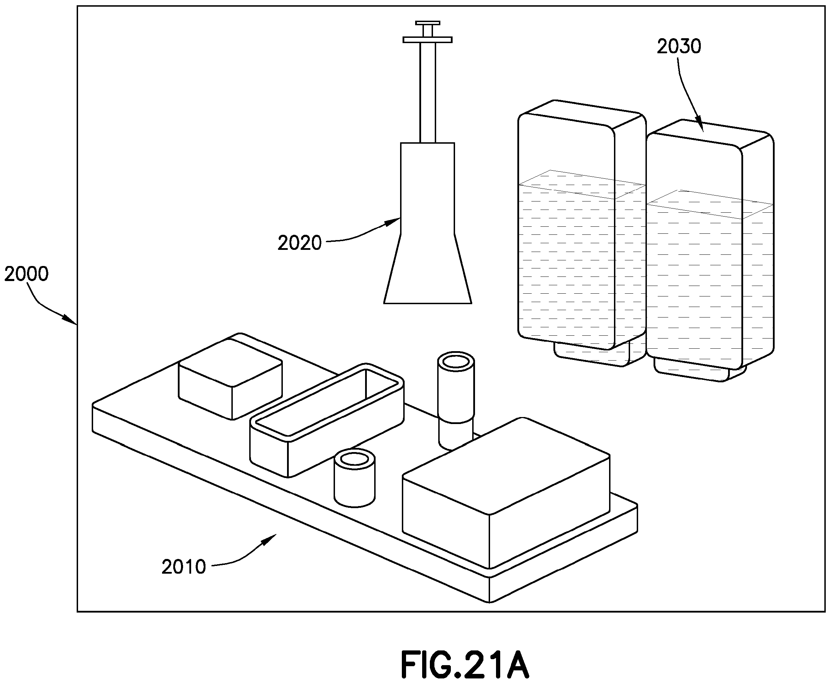

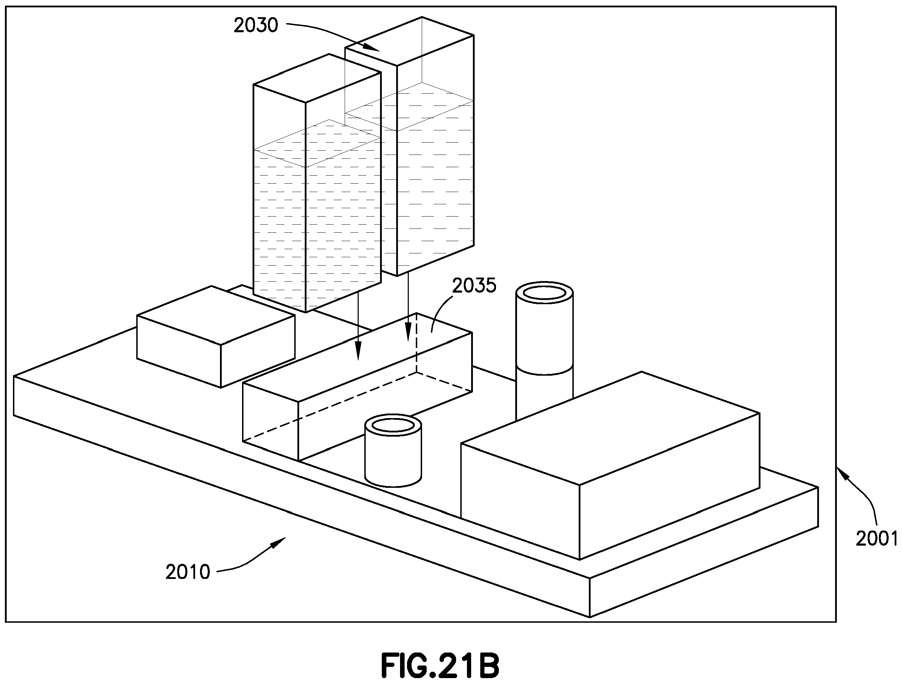

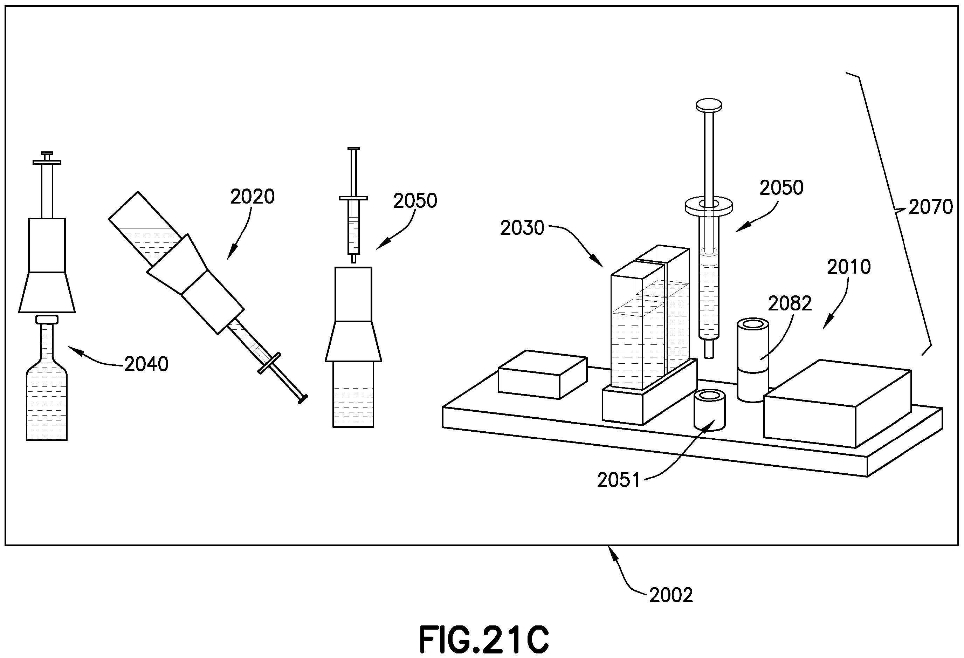

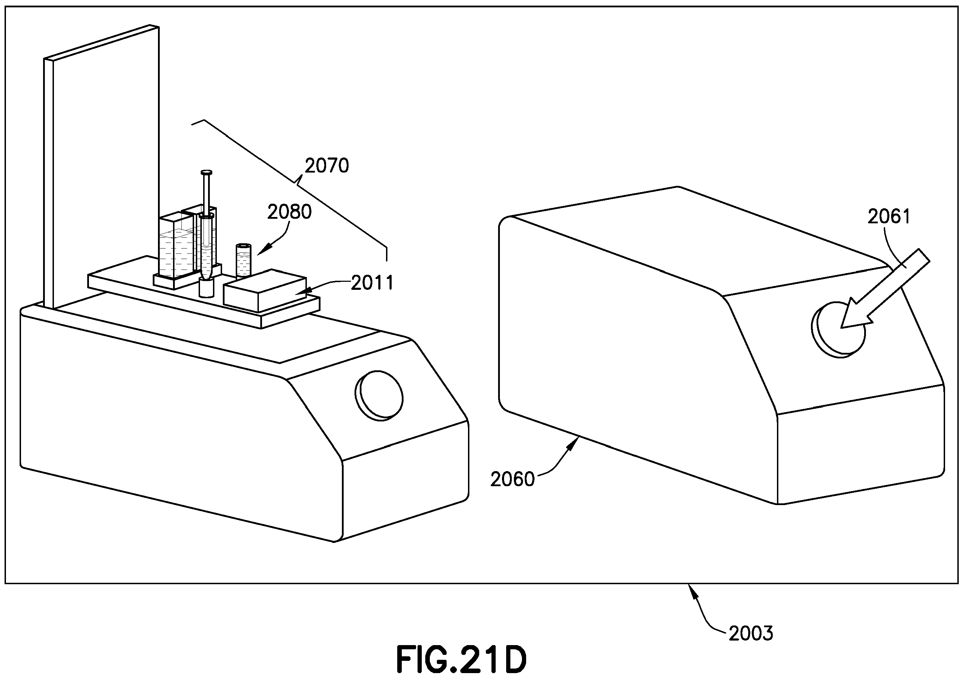

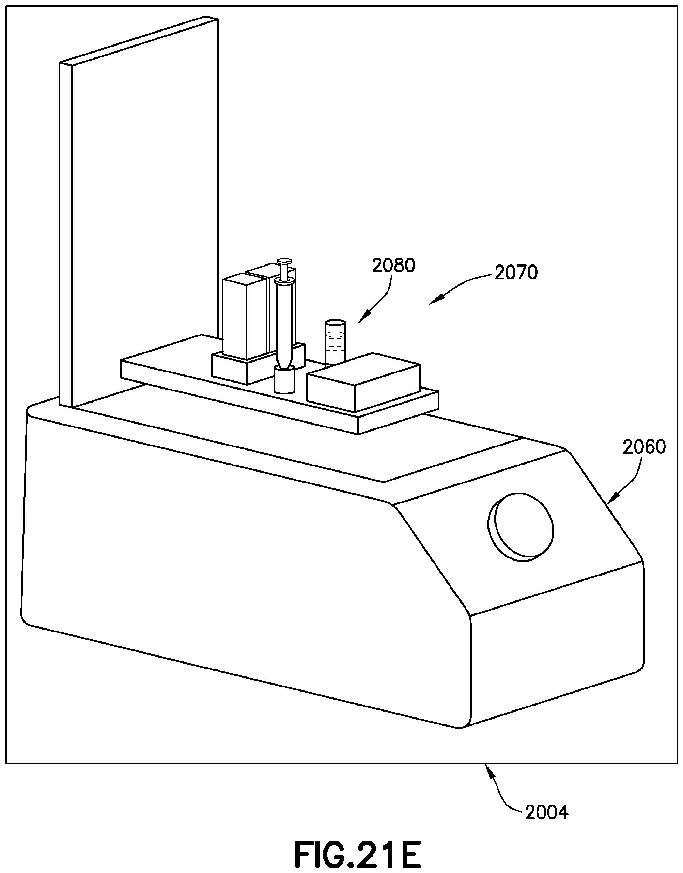

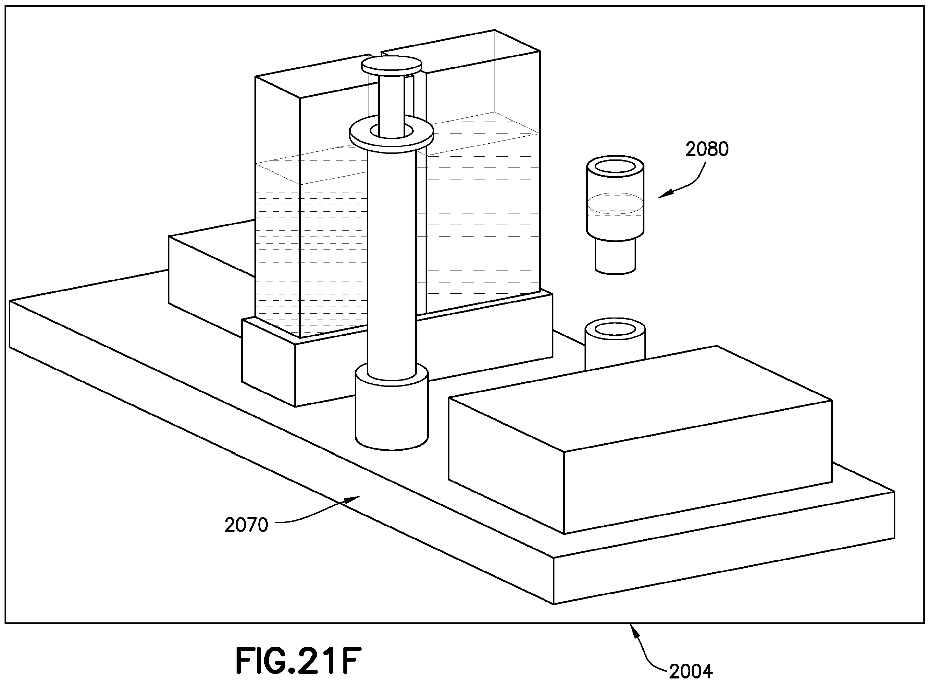

FIGs. 21A-F illustrate an optional workflow for processing a PBC to isolate, purify and or concentrate pathogens in the PBC. Step 2000 (FIG. 21A) illustrates the disposable components; a cartridge 2010 containing the tangential flow membrane device, a pre-filled reagent vessel 2030 required for processing the PBC and an apparatus 2020 that is used to obtain an aliquot of the PBC for processing. The disposables are removed from any packaging prior to use. Step 2001 (FIG. 21B) illustrates the assembly of the reagent vessel 2030 to a tray 2035 in the cartridge 2010. Such assembly puts the reagent vessel 2030 into fluid communication with the cartridge 2010. In step 2002 (FIG. 21C), the apparatus 2020 is used to draw the required volume from PBC culture bottle 2040 in a safety cabinet (not shown). The apparatus 2020 includes a removable vessel 2050, illustrated as a syringe, that is then connected to the cartridge 2010 via a port 2051 therein. The port 2051 provides a fluid-tight connection with the syringe. In one example the port 2051 has a luer lock for a fluid-tight connection with the syringe 2050. An illustrated example of a collection device that couples with a syringe to obtain an aliquot of PBC from a culture bottle is disclosed in US Provisional Application No. 62/883,427 filed August 6, 2019 and US Provisional Application No. 62/907,060 filed September 27, 2019. Both applications are commonly assigned with the present application. Step 2003 (FIG. 21D) illustrates the assembled processing device 2070 loaded into an instrument 2060, which is opened and closed using switch 2061. During processing, the sample injected into the cartridge 2010 is forced through the tangential flow membrane device (located in housing 2011) which separates components of interest (e.g. pathogens) from other sample constituents. The components of interest are collected into vial 2080 which is disposed in a receptacle 2082 that is in fluid communication with the permeate side of the track etched membrane disposed inside the cartridge 2010. Once the processing is complete, the assembled processing device 2070 is removed from the instrument 2060 as shown in step 2004 (FIG. 21E). Finally, in step 2005 (FIG. 21F), the isolated, purified and/or concentrated component of interest (e.g. pathogens) which have been collected into vial 2080 is separated from the device 2070 which is disposable and is therefore discarded. The vial 2080 is removed from fluid attachment with the device 2070 in a biosafety cabinet (not shown).

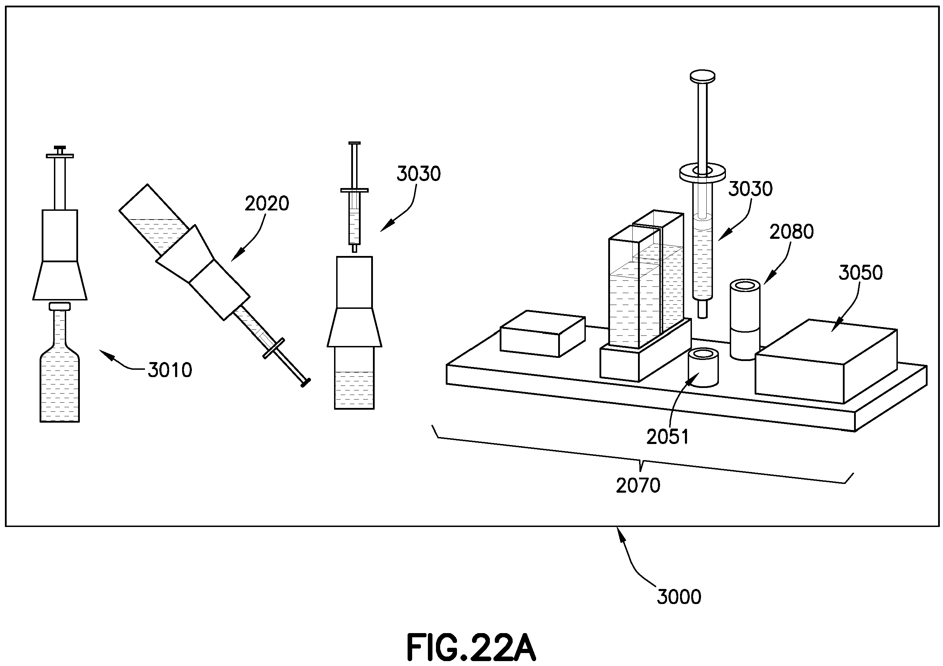

FIGs. 22A-B illustrate different ways in which the PBC sample may be introduced into the assembled processing device 2070. In 3000 (FIG. 22A), an apparatus 3020 is used to aliquot the required volume of PBC (e.g. 10 mL) from the culture bottle 3010 into a vessel 3030. As in the previous example, aliquoting is performed in a biosafety cabinet (not shown). In this example, the vessel 3030 is a syringe but any alternative vessel with the ability to provide a sub-atmospheric draw force to collect an aliquot of PBC from a culture bottle, for example a BD Vacutainer^{®}, is contemplated as suitable. The vessel 3030 is then fluidically connected to the assembled cartridge 2070, via port 2051. The cartridge contains the tangential flow membrane device in housing 3050. In a different optional configuration 3001 (FIG. 22B), the PBC bottle 3010 is directly loaded onto the cartridge 2070 containing the tangential flow membrane device 3050 via the port 3055. During the processing, the required PBC volume is drawn from the PBC bottle 3010 by the processing device 2070 and into the membrane device disposed in housing 3050 by the instrument (not shown). As in the prior embodiment, the target sample fraction dispenses from the membrane device 3050 and through the cartridge 2010 and into the vial 2080.

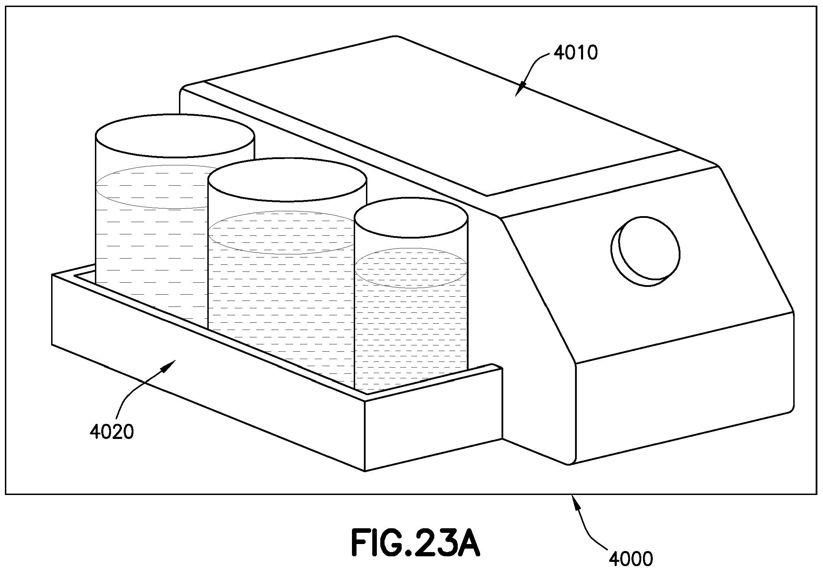

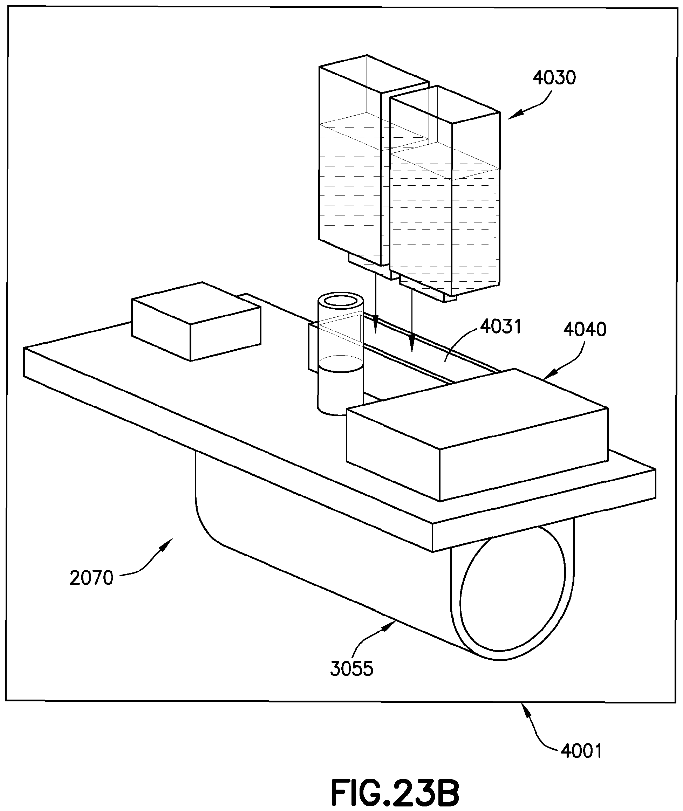

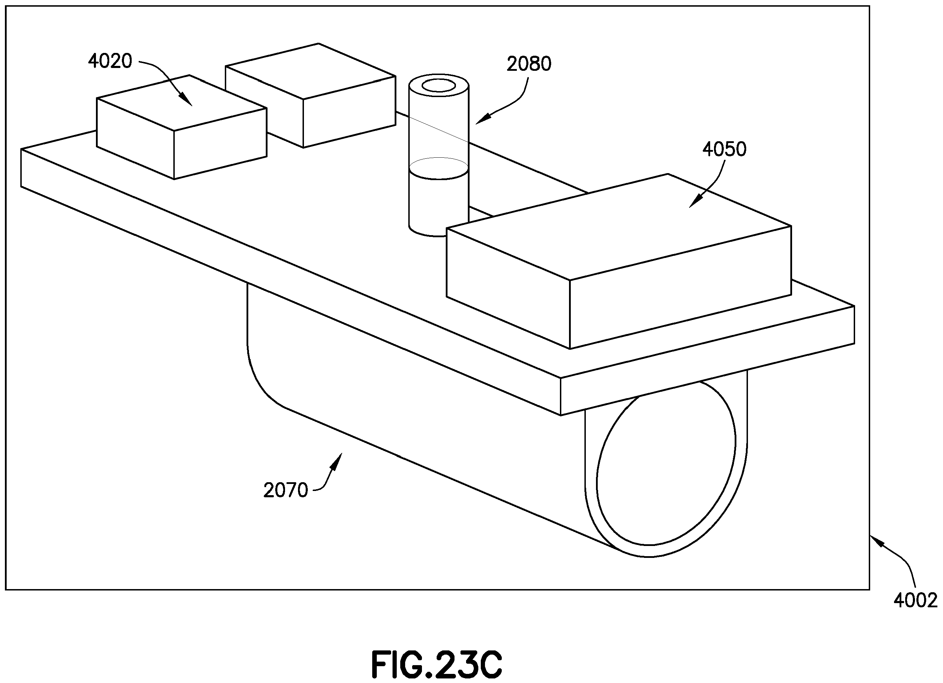

FIG. 23A-C illustrates an optional way in which the reagents may be supplied. In option 4000 (FIG. 23A), the reagents 4020 are loaded onto the instrument 4010 and the instrument 4010 distributes the reagents 4020 to the processing cartridge (not shown). The instrument 4010 stores regents to process one or more PBC samples. In option 4001 (FIG. 23B), a pre-filled reagent vessel 4030 is attached to the cartridge 2070 containing the tangential flow membrane device 4040 by receiving them into tray 4031. In option 4001(FIG. 23 B), the PBC bottle (not shown) is fluidically connected to the cartridge 2070 via port 3055. In option 4002 (FIG. 23C), the reagents 4021 are pre-filled in the cartridge 2070 containing the tangential flow membrane device in housing 4050.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to require any order but may be utilized to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously. In this disclosure, when a range is provided it is understood that the range may include any value within the range as well as the limits. Approximate values may be utilized and will be understood to include all values significantly near a stated value with reference to the stated value's significant digits.

It is therefore to be understood that numerous modifications may be made to the illustrative examples and that other arrangements may be devised without departing from the scope of the technology, the invention being defined by the appended claims.

## Claims

1. Apparatus for extracting constituents from a biological sample for concentrating a desired constituent of the biological sample, the apparatus comprising:
a plurality of membrane devices, wherein each of the plurality of membrane devices (600) comprises:
a membrane (630) having a first side, a second side, a first end and a second end, the membrane having a plurality of pores having a pore size to selectively permit one or more constituents of the biological sample to pass through the membrane from the first side to the second side while retaining other constituents of the biological sample at the first side;
an input chamber (610) having an inlet proximate to the first end and an outlet proximate to the second end, the input chamber (610) configured at the first side of the membrane (630) to permit (a) a tangential flow of the biological sample along a first surface of the membrane (630) at the first side from the inlet to the outlet, and (b) a trans-membrane passing of the one or more constituents of the biological sample from the first side to the second side;
an output chamber (620)
configured at a second surface of the membrane (630) at the second side and configured to receive the one or more constituents of the biological sample that pass through the membrane (630); and
a pressure source coupled to the inlet of the input chamber (610), wherein the plurality of membrane devices are collectively configured to isolate a desired passband of constituents of the biological sample, **characterized in that** each membrane device (600) further comprises an additional pressure source coupled to the outlet of the input chamber (610) and configured to induce the tangential flow of the biological sample along the first surface of the membrane (630), wherein the tangential flow is a reciprocating flow.

2. The apparatus of claim 1 wherein the membrane (630) comprises a track etched membrane formed of any of a polyester material, a polyimide material, a polypropylene material and a polycarbonate material.

3. The apparatus of claim 1, wherein the pore size of the plurality of pores is an opening width chosen to isolate constituents of the biological sample in a size range of about 0.01 to 8.0 micrometers (µm) and optionally wherein the pore size of the membrane is substantially uniform for the membrane

4. The apparatus of any one of claims 1 to 3, wherein the pore size of the plurality of pores of the membrane of the plurality of membrane devices (600) is about 0.03 to 8.0 µm and optionally, wherein the pore size of the plurality of pores of the membrane of the plurality of membrane devices (600) is about 0.03 to 0.40 µm.

5. The apparatus of claim 1, wherein the pressure source comprises one of a pump, a plunger, a syringe, a peristaltic pump, a pressurized chamber, a de-pressurized chamber, or a vacuum.

6. The apparatus of any one of claims 1 to 2 further comprising a pressure source coupled to an outlet of the output chamber and configured to induce the trans-membrane passing of the one or more constituents.

7. The apparatus of any one of claims 1 or 6, wherein the pressure source coupled to the outlet of the output chamber comprises one of a pump, a plunger, a syringe, a peristaltic pump, a de-pressurized chamber, or a vacuum.

8. The apparatus of any one of claims 1 to 2, wherein the apparatus comprises an integrated vessel including a first membrane device of the plurality of membrane devices.

9. The apparatus of claim 8, wherein the integrated vessel includes a sample container coupled to the input chamber (610) of the first membrane device, the sample container comprising at least one of an outlet for providing the biological sample to the first membrane device or an inlet for receiving retentate from the first membrane device (600);
wherein the integrated vessel optionally further includes a collection container (1514) coupled to the output chamber of the first membrane device, the collection container comprising at least one of an inlet or an outlet for providing a rinse to the first membrane device and optionally receiving permeate from the first membrane device (600).

10. A method for extracting constituents from a biological sample using the apparatus from any of claims 1 to 9, the method comprising:
in a first extracting process, passing, by a pressure force, one or more constituents of the biological sample through the membrane of a first membrane device from the first side of the membrane to the second side of the membrane while retaining other constituents of the biological sample at the first side of the membrane and while generating the tangential flow of the biological sample along the surface of the membrane at the first side of the membrane wherein the tangential flow is the reciprocating flow induced by the pressure source of the first membrane device and the additional pressure source of the first membrane device, wherein the first membrane has a first characteristic pore size; and
in a second extracting process, passing by a pressure force, one or more additional constituents of the one or more constituents through the membrane of a second membrane device from the first side of the membrane to the second side of the membrane while retaining some of the one or more constituents at the first side of the membrane and while generating the tangential flow of the one or more constituents along the surface of the second membrane at the first side of the membrane, wherein the membrane is a second membrane and wherein the second membrane has a second characteristic pore size that is different than the first characteristic pore size,
whereby the first extracting process and the second extracting process isolate a desired passband of constituents of the biological sample from the biological sample.

11. The method of claim 10, wherein the biological sample is selected from the group consisting of positive blood culture, whole blood, urine, cerebrospinal fluid, saliva, respiratory samples, and wounds wherein the biological sample is diluted or undiluted, and the wherein desired passband of constituents is an isolated bacteria from the biological sample.

12. The method of claim 10, wherein the characteristic pore size of the first membrane is about 3.0 µm to 5 µm and the characteristic pore size of the second membrane is about 0.40 µm to about 1 µm.

13. The method of claim 10, wherein the biological sample is one of blood or urine, and wherein the desired passband of constituents is isolated exosomes from the biological sample.

14. The method of claim 13, wherein the characteristic pore size of the first membrane is about 0.40 µm to about 1 µm and the characteristic pore size of the second membrane is about 0.050 µm or less

15. The method of any one of claims 13 or 14, further comprising:
in a third extracting process, passing by a pressure force, a plurality of constituents of the biological sample through the membrane of a third membrane device from the first side of the membrane to the second side of the membrane while retaining some of the biological sample at the first side of the membrane and while generating the tangential flow of the biological sample along the surface of the membrane at the first side of the membrane, wherein the membrane is a third membrane and wherein the third membrane has a characteristic pore size that is about 3.0 µm.

16. The method of claim 15 further comprising
in a fourth extracting process, passing by a pressure force, some constituents of the retained one or more constituents from the first side of the second membrane, the passing being through the membrane of a fourth membrane device from the first side of the membrane to the second side of the membrane while retaining some of the retained one or more constituents, and while generating the tangential flow of the retained one or more constituents along the surface of the membrane at the first side of the membrane, wherein the membrane is a fourth membrane and wherein the fourth membrane has a characteristic pore size that is about 0.10 or 0.20 µm.

## Patentansprüche

1. Vorrichtung zur Extraktion von Bestandteilen aus einer biologischen Probe, um einen gewünschten Bestandteil der biologischen Probe zu konzentrieren, wobei die Vorrichtung aufweist:
eine Vielzahl von Membranvorrichtungen, wobei jede der Vielzahl von Membranvorrichtungen (600) aufweist:
einen Membran (630) mit einer ersten Seite, einer zweiten Seite, einem ersten Ende und einem zweiten Ende, wobei die Membran eine Vielzahl von Poren mit einer Porengröße aufweist, um einem oder mehreren Bestandteilen der biologischen Probe selektiv zu ermöglichen, durch die Membran von der ersten Seite zur zweiten Seite zu passieren, während andere Bestandteile der biologischen Probe auf der ersten Seite zurückgehalten werden;
eine Eingangskammer (610) mit einem Einlass in der Nähe des ersten Endes und einem Auslass in der Nähe des zweiten Endes, wobei die Eingangskammer (610) auf der ersten Seite der Membran (630) dazu ausgebildet ist, (a) eine Tangentialströmung der biologischen Probe entlang einer ersten Fläche der Membran (630) auf der ersten Seite vom Einlass zum Auslass zu ermöglichen, und (b) eine Transmembranpassage des einen oder der mehreren Bestandteile der biologischen Probe von der ersten Seite zur zweiten Seite zu ermöglichen;
eine Ausgangskammer (620), die an einer zweiten Fläche der Membran (630) auf der zweiten Seite ausgebildet ist und dazu ausgebildet ist, den einen oder die mehreren Bestandteile der biologischen Probe aufzunehmen, die durch die Membran (630) passieren; und
eine Druckquelle, die mit dem Einlass der Eingangskammer (610) gekoppelt ist, wobei die Vielzahl von Membranvorrichtungen gemeinsam dazu ausgebildet sind, einen gewünschten Durchlassbereich von Bestandteilen der biologischen Probe zu isolieren, **dadurch gekennzeichnet, dass** jede Membranvorrichtung (600) ferner eine zusätzliche Druckquelle aufweist, die mit dem Auslass der Eingangskammer (610) gekoppelt und dazu ausgebildet ist, die Tangentialströmung der biologischen Probe entlang der ersten Fläche der Membran (630) zu veranlassen, wobei die Tangentialströmung eine hin- und hergehende Strömung ist.

2. Vorrichtung nach Anspruch 1, wobei die Membran (630) eine Kernspurmembran aufweist, die aus einem aus einem Polyestermaterial, einem Polyimidmaterial, einem Polyproylenmaterial und einem Polycarbonatmaterial ausgebildet ist.

3. Vorrichtung nach Anspruch 1, wobei die Porengröße der Vielzahl von Poren eine Öffnungsbreite ist, die gewählt wird, um Bestandteile der biologischen Probe in einem Größenbereich von etwa 0,01 bis 8,0 Mikrometern (µm) zu isolieren, und wobei optional die Porengröße der Membran im Wesentlichen einheitlich für die Membran ist.

4. Vorrichtung nach einem der Ansprüche 1 bis 3, wobei die Porengröße der Vielzahl von Poren der Membran der Vielzahl von Membranvorrichtungen (600) etwa 0,03 bis 8,0 µm beträgt, und wobei optional die Porengröße der Vielzahl von Poren der Membran der Vielzahl von Membranvorrichtungen (600) etwa 0,03 bis 0,40 µm beträgt.

5. Vorrichtung nach Anspruch 1, wobei die Druckquelle eines aus einer Pumpe, einem Kolben, einer Spritze, einer Schlauchpumpe, einer Druckkammer, einer Druckentlastungskammer, oder einem Vakuum aufweist.

6. Vorrichtung nach einem der Ansprüche 1 bis 2, die ferner eine Druckquelle aufweist, die mit einem Auslass der Ausgangskammer gekoppelt und dazu ausgebildet ist, den Transmembrandurchgang des einen oder der mehreren Bestandteile zu veranlassen.

7. Vorrichtung nach einem der Ansprüche 1 oder 6, wobei die Druckquelle, die mit dem Auslass der Ausgangskammer gekoppelt ist, eines aus einer Pumpe, einem Kolben, einer Spritze, einer Schlauchpumpe, einer Druckkammer, einer Druckentlastungskammer, oder einem Vakuum aufweist.

8. Vorrichtung nach einem der Ansprüche 1 bis 2, wobei die Vorrichtung ein integriertes Gefäß aufweist, das eine erste Membranvorrichtung der Vielzahl von Membranvorrichtungen aufweist.

9. Vorrichtung nach Anspruch 8, wobei das integrierte Gefäß einen Probenbehälter aufweist, der mit der Eingangskammer (610) der ersten Membranvorrichtung gekoppelt ist, wobei der Probenbehälter einen Auslass zum Bereitstellen der biologischen Probe an die erste Membranvorrichtung und/oder einen Einlass zum Aufnehmen von Retentat von der ersten Membranvorrichtung (600) aufweist;
wobei das integrierte Gefäß optional ferner einen Sammelbehälter (1514) aufweist, der mit der Ausgangskammer der ersten Membranvorrichtung gekoppelt ist, wobei der Sammelbehälter einen Einlass und/oder einen Auslass zum Bereitstellen einer Spülung an die erste Membranvorrichtung und optional zum Aufnehmen von Permeat von der ersten Membranvorrichtung (600) aufweist.

10. Verfahren zur Extraktion von Bestandteilen aus einer biologischen Probe mittels der Vorrichtung nach einem der Ansprüche 1 bis 9, wobei das Verfahren umfasst:
in einem ersten Extraktionsprozess, Durchleiten von einem oder mehreren Bestandteilen der biologischen Probe durch eine Druckkraft durch die Membran einer ersten Membranvorrichtung von der ersten Seite der Membran zur zweiten Seite der Membran, während andere Bestandteile der biologischen Probe auf der ersten Seite der Membran zurückgehalten werden und während die Tangentialströmung der biologischen Probe entlang der Fläche der Membran auf der ersten Seite der Membran erzeugt wird, wobei die Tangentialströmung die hin- und hergehende Strömung ist, die von der Druckquelle der ersten Membranvorrichtung und der zusätzlichen Druckquelle der ersten Membranvorrichtung veranlasst wird, wobei die erste Membran eine erste charakteristische Porengröße aufweist; und
in einem zweiten Extraktionsprozess, Durchleiten eines oder mehrerer zusätzlicher Bestandteile des einen oder der mehreren Bestandteile durch eine Druckkraft durch die Membran einer zweiten Membranvorrichtung von der ersten Seite der Membran zur zweiten Seite der Membran, während ein Teil des einen oder der mehreren Bestandteile auf der ersten Seite der Membran zurückgehalten werden und während die Tangentialströmung des einen oder der mehreren Bestandteile entlang der Fläche der zweiten Membran auf der ersten Seite der Membran erzeugt wird, wobei die Membran eine zweite Membran ist, und wobei die zweite Membran eine zweite charakteristische Porengröße aufweist, die sich von der ersten charakteristischen Porengröße unterscheidet,
wobei der erste Extraktionsprozess und der zweite Extraktionsprozess einen gewünschten Durchlassbereich der Bestandteile der biologischen Probe von der biologischen Probe isolieren.

11. Verfahren nach Anspruch 10, wobei die biologische Probe ausgewählt wird aus der Gruppe bestehend aus einer positiven Blutkultur, Vollblut, Urin, Zerebrospinalflüssigkeit, Speichel, respiratorischen Proben, und Wunden, wobei die biologische Probe verdünnt oder unverdünnt ist, und wobei der gewünschte Durchlassbereich von Bestandteilen ein isoliertes Bakterium aus der biologischen Probe ist.

12. Verfahren nach Anspruch 10, wobei die charakteristische Porengröße der ersten Membran etwa 3,0 µm bis 5 µm beträgt und die charakteristische Porengröße der zweiten Membran am 0,40 µm bis etwa 1 µm beträgt.

13. Verfahren nach Anspruch 10, wobei die biologische Probe eines aus Blut oder Urin ist, und wobei der gewünschte Durchlassbereich von Bestandteilen isolierte Exosomen aus der biologischen Probe sind.

14. Verfahren nach Anspruch 13, wobei die charakteristische Porengröße der ersten Membran etwa 0,40 µm bis etwa 1 µm beträgt und die charakteristische Porengröße der zweiten Membran etwa 0,050 µm oder weniger beträgt.

15. Verfahren nach einem der Ansprüche 13 oder 14, das ferner umfasst:
in einem dritten Extraktionsprozess, Durchleiten einer Vielzahl von Bestandteilen der biologischen Probe durch Druckkraft durch die Membran einer dritten Membranvorrichtung von der ersten Seite der Membran zur zweiten Seite der Membran, während ein Teil der biologischen Probe auf der erste Seite der Membran zurückgehalten wird und während die Tangentialströmung der biologischen Probe entlang der Fläche der Membran auf der ersten Seite der Membran erzeugt wird, wobei die Membran eine dritte Membran ist, und wobei die dritte Membran eine charakteristische Porengröße hat, die etwa 3,0 µm beträgt.

16. Verfahren nach Anspruch 15, das ferner umfasst:
in einem vierten Extraktionsprozess, Durchleiten eines Teils der Bestandteile des/der zurückgehaltenen einen oder mehreren Bestandteile von der ersten Seite der zweiten Membran, wobei das Durchleiten durch die Membran einer vierten Membranvorrichtung von der ersten Seite der Membran zur zweiten Seite der Membran erfolgt, während ein Teil des/der zurückgehaltenen einen oder mehreren Bestandteile zurückgehalten wird, und während die Tangentialströmung des/der zurückgehaltenen einen oder mehreren Bestandteile entlang der Fläche der Membran auf der ersten Seite der Membran erzeugt wird, wobei die Membran eine vierte Membran ist, und wobei die vierte Membran eine charakteristische Porengröße aufweist, die etwa 0,10 oder 0,20 µm beträgt.

## Revendications

1. Appareil pour extraire des constituants d'un échantillon biologique pour concentrer un constituant souhaité de l'échantillon biologique, l'appareil comprenant :
une pluralité de dispositifs à membrane, dans lequel chacun de la pluralité de dispositifs à membrane (600) comprend :
une membrane (630) ayant un premier côté, un second côté, une première extrémité et une seconde extrémité, la membrane ayant une pluralité de pores ayant une taille de pore pour permettre sélectivement à un ou plusieurs constituants de l'échantillon biologique de traverser la membrane du premier côté au second côté tout en conservant d'autres constituants de l'échantillon biologique du premier côté ;
une chambre d'entrée (610) ayant une entrée à proximité de la première extrémité et une sortie à proximité de la seconde extrémité, la chambre d'entrée (610) étant configurée sur le premier côté de la membrane (630) pour permettre (a) un écoulement tangentiel de l'échantillon biologique le long d'une première surface de la membrane (630) sur le premier côté de l'entrée à la sortie, et (b) un passage transmembranaire du ou des plusieurs constituants de l'échantillon biologique du premier côté au second côté ;
une chambre de sortie (620) configurée au niveau d'une seconde surface de la membrane (630) sur le second côté et configurée pour recevoir le ou les plusieurs constituants de l'échantillon biologique qui traversent la membrane (630) ; et
une source de pression couplée à l'entrée de la chambre d'entrée (610), dans lequel la pluralité de dispositifs à membrane sont configurés collectivement pour isoler une bande passante souhaitée de constituants de l'échantillon biologique, **caractérisé en ce que** chaque dispositif à membrane (600) comprend en outre une source de pression supplémentaire couplée à la sortie de la chambre d'entrée (610) et configurée pour induire l'écoulement tangentiel de l'échantillon biologique le long de la première surface de la membrane (630), dans lequel l'écoulement tangentiel est un écoulement alternatif.

2. Appareil de la revendication 1, dans lequel la membrane (630) comprend une membrane à traces attaquées formée d'un matériau parmi un matériau polyester, un matériau polyimide, un matériau polypropylène et un matériau polycarbonate.

3. Appareil de la revendication 1, dans lequel la taille de pore de la pluralité de pores est une largeur d'ouverture choisie pour isoler des constituants de l'échantillon biologique dans une plage de tailles d'environ 0,01 à 8,0 micromètres (µm) et optionnellement dans lequel la taille de pore de la membrane est sensiblement uniforme pour la membrane.

4. Appareil de l'une quelconque des revendications 1 à 3, dans lequel la taille de pore de la pluralité de pores de la membrane de la pluralité de dispositifs à membrane (600) est d'environ 0,03 à 8,0 µm et optionnellement, dans lequel la taille de pore de la pluralité de pores de la membrane de la pluralité de dispositifs à membrane (600) est d'environ 0,03 à 0,40 µm.

5. Appareil de la revendication 1, dans lequel la source de pression comprend l'un parmi une pompe, un piston, une seringue, une pompe péristaltique, une chambre sous pression, une chambre dépressurisée ou un vide.

6. Appareil de l'une quelconque des revendications 1 et 2, comprenant en outre une source de pression couplée à une sortie de la chambre de sortie et configurée pour induire le passage transmembranaire du ou des plusieurs constituants.

7. Appareil de l'une quelconque des revendications 1 ou 6, dans lequel la source de pression couplée à la sortie de la chambre de sortie comprend l'un parmi une pompe, un piston, une seringue, une pompe péristaltique, une chambre dépressurisée ou un vide.

8. Appareil de l'une quelconque des revendications 1 et 2, dans lequel l'appareil comprend une cuve intégrée comprenant un premier dispositif à membrane de la pluralité de dispositifs à membrane.

9. Appareil de la revendication 8, dans lequel la cuve intégrée comprend un récipient à échantillon couplé à la chambre d'entrée (610) du premier dispositif à membrane, le récipient à échantillon comprenant au moins l'une parmi une sortie pour fournir l'échantillon biologique au premier dispositif à membrane ou une entrée pour recevoir un rétentat du premier dispositif à membrane (600) ;
dans lequel la cuve intégrée comprend en outre optionnellement un récipient de collecte (1514) couplé à la chambre de sortie du premier dispositif à membrane, le récipient de collecte comprenant au moins l'une parmi une entrée ou une sortie pour fournir un rinçage au premier dispositif à membrane et optionnellement recevoir un perméat du premier dispositif à membrane (600).

10. Procédé d'extraction de constituants d'un échantillon biologique en utilisant l'appareil de l'une des revendications 1 à 9, le procédé comprenant :
dans un premier processus d'extraction, le passage, par une force de pression, d'un ou de plusieurs constituants de l'échantillon biologique à travers la membrane d'un premier dispositif à membrane du premier côté de la membrane au second côté de la membrane tout en conservant d'autres constituants de l'échantillon biologique du premier côté de la membrane et tout en générant l'écoulement tangentiel de l'échantillon biologique le long de la surface de la membrane sur le premier côté de la membrane, dans lequel l'écoulement tangentiel est l'écoulement alternatif induit par la source de pression du premier dispositif à membrane et la source de pression supplémentaire du premier dispositif à membrane,
dans lequel la première membrane présente une première taille de pore caractéristique ; et
dans un deuxième processus d'extraction, le passage par une force de pression, d'un ou de plusieurs constituants supplémentaires du ou des plusieurs constituants à travers la membrane d'un deuxième dispositif à membrane du premier côté de la membrane au second côté de la membrane tout en conservant une partie du ou des plusieurs constituants du premier côté de la membrane et tout en générant l'écoulement tangentiel du ou des plusieurs constituants le long de la surface de la deuxième membrane sur le premier côté de la membrane, dans lequel la membrane est une deuxième membrane et dans lequel la deuxième membrane présente une seconde taille de pore caractéristique qui est différente de la première taille de pore caractéristique,
moyennant quoi le premier processus d'extraction et le deuxième processus d'extraction isolent une bande passante souhaitée de constituants de l'échantillon biologique de l'échantillon biologique.

11. Procédé de la revendication 10, dans lequel l'échantillon biologique est choisi dans le groupe constitué d'une hémoculture positive, du sang total, de l'urine, du liquide céphalorachidien, de la salive, d'échantillons respiratoires et de plaies où l'échantillon biologique est dilué ou non dilué, et dans lequel la bande passante souhaitée des constituants est une bactérie isolée de l'échantillon biologique.

12. Procédé de la revendication 10, dans lequel la taille de pore caractéristique de la première membrane est d'environ 3,0 µm à 5 µm et la taille de pore caractéristique de la deuxième membrane est d'environ 0,40 µm à environ 1 µm.

13. Procédé de la revendication 10, dans lequel l'échantillon biologique est choisi parmi du sang ou de l'urine, et dans lequel la bande passante souhaitée des constituants est constituée d'exosomes isolés de l'échantillon biologique.

14. Procédé de la revendication 13, dans lequel la taille de pore caractéristique de la première membrane est d'environ 0,40 µm à environ 1 µm et la taille de pore caractéristique de la deuxième membrane est inférieure ou égale à environ 0,050 µm.

15. Procédé de l'une quelconque des revendications 13 ou 14, comprenant en outre :
dans un troisième processus d'extraction, le passage par une force de pression, d'une pluralité de constituants de l'échantillon biologique à travers la membrane d'un troisième dispositif à membrane du premier côté de la membrane au second côté de la membrane tout en conservant une partie de l'échantillon biologique sur le premier côté de la membrane et tout en générant l'écoulement tangentiel de l'échantillon biologique le long de la surface de la membrane sur le premier côté de la membrane, dans lequel la membrane est une troisième membrane et dans lequel la troisième membrane présente une taille de pore caractéristique qui est d'environ 3,0 µm.

16. Procédé de la revendication 15 comprenant en outre
dans un quatrième processus d'extraction, le passage par une force de pression, d'une partie de constituants du ou des plusieurs constituants conservés depuis le premier côté de la deuxième membrane, le passage s'effectuant à travers la membrane d'un quatrième dispositif à membrane du premier côté de la membrane au second côté de la membrane tout en conservant une partie du ou des plusieurs constituants conservés, et tout en générant l'écoulement tangentiel du ou des plusieurs constituants conservés le long de la surface de la membrane sur le premier côté de la membrane, dans lequel la membrane est une quatrième membrane et dans lequel la quatrième membrane présente une taille de pore caractéristique qui est d'environ 0,10 ou 0,20 µm.
